(19) Europäisches Patentamt
European Patent Office
Office européen des brevets

(11) Veröffentlichungsnummer: **0 436 680 B1**

# (12) EUROPÄISCHE PATENTSCHRIFT

(45) Veröffentlichungstag der Patentschrift: **07.09.94**

(51) Int. Cl.5: **C07D 239/54**, C07D 239/70, C07D 239/96, A01N 43/54

(21) Anmeldenummer: **90909122.5**

(22) Anmeldetag: **27.06.90**

(86) Internationale Anmeldenummer:
**PCT/CH90/00153**

(87) Internationale Veröffentlichungsnummer:
**WO 91/00278 (10.01.91 91/02)**

## (54) HETEROCYCLISCHE VERBINDUNGEN.

(30) Priorität: **29.06.89 CH 2450/89**

(43) Veröffentlichungstag der Anmeldung:
**17.07.91 Patentblatt 91/29**

(45) Bekanntmachung des Hinweises auf die Patenterteilung:
**07.09.94 Patentblatt 94/36**

(84) Benannte Vertragsstaaten:
**AT BE CH DE DK ES FR GB IT LI LU NL SE**

(56) Entgegenhaltungen:
**EP-A- 0 195 346**
**EP-A- 0 255 047**
**EP-A- 0 260 621**

(73) Patentinhaber: **CIBA-GEIGY AG**
**Klybeckstrasse 141**
**CH-4002 Basel (CH)**

(72) Erfinder: **SUCHY, Milos**
**Grossplatzstrasse 3**
**D-8122 Pfaffhausen (CH)**
Erfinder: **WINTERNITZ, Paul**
**Am Pfisterhölzli 50**
**D-8606 Greifensee (CH)**
Erfinder: **ZELLER, Martin**
**Kronengasse 7**
**CH-5400 Baden (CH)**

**EP 0 436 680 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft heterocyclische Verbindungen, und zwar 3-Aryluracile der allgemeinen Formel

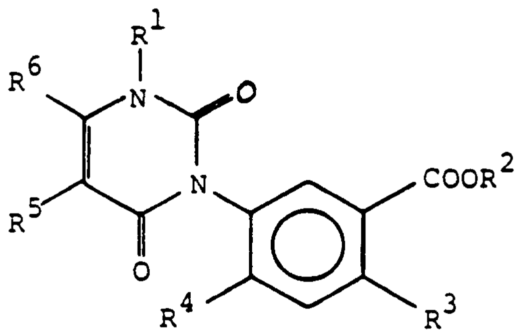

I

worin

R[1] Wasserstoff, $C_{1-4}$-Alkyl. $C_{3\ oder\ 4}$-Alkenyl. $C_{3\ oder\ 4}$-Alkinyl oder $C_{1-4}$-Halogenalkyl,

R[2] $C_{1-6}$-Alkylsulfonyl-$C_{1-4}$-alkyl, Di($C_{1-6}$-alkyl)phosphono-$C_{1-4}$-alkyl. Tri($C_{1-6}$-alkyl)silyl-$C_{1-4}$-alkyl, $C_{2-7}$-Alkanoyl-$C_{1-4}$-alkyl, Carboxy-$C_{1-4}$-alkyl, ($C_{1-6}$-Alkoxy)carbonyl-$C_{1-4}$-alkyl, {[$C_{3-9}$-(α-Alkylalkyliden)]iminooxy-($C_{1-6}$-alkoxy)carbonyl}-$C_{1-4}$-alkyl, ($C_{3-6}$-Alkenyloxy)carbonyl-$C_{1-4}$-alkyl, ($C_{3-6}$-Alkinyloxy)carbonyl-$C_{1-4}$-alkyl, ($C_{3-8}$-Cycloalkyloxy)-carbonyl-$C_{1-4}$-alkyl, Phenoxy-$C_{1-4}$-alkyl, $C_{3-6}$-Alkenyloxy-$C_{1-4}$-alkyl, $C_{3-8}$-Cycloalkenyloxy-$C_{1-4}$-alkyl, $C_{5-8}$-Cycloalkenyl, $C_{6-8}$-Bicycloalkyl, $C_{6-8}$-Bicycloalkenyl, $C_{2-7}$-Cyanoalkyl oder $C_{3-6}$-Alkinyloxy-$C_{1-4}$-alkyl,

R[3] Halogen oder Cyano,

R[4] Wasserstoff oder Halogen,

R[5] Wasserstoff, Fluor oder $C_{1-4}$-Alkyl und

R[6] $C_{1-4}$-Alkyl oder $C_{1-4}$-Halogenalkyl bedeuten, oder

R[5] und R[6] zusammen Tri- oder Tetramethylen bilden,

und die entsprechenden Enoläther derjenigen Verbindungen der Formel I, in denen $R^1$ von Wasserstoff oder $C_{1-4}$-Halogenalkyl verschieden ist, sowie Salze derjenigen Verbindungen der Formel I, in denen $R^1$ Wasserstoff bedeutet.

Unter den oben erwähnten Enoläthern sind also die Verbindungen der Formel

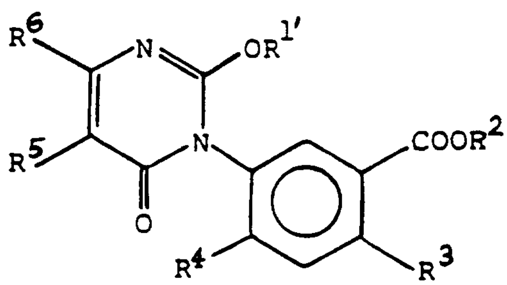

Ia

worin $R^{1'}$ $C_{1-4}$-Alkyl, $C_{3\ oder\ 4}$-Alkenyl oder $C_{3\ oder\ 4}$-Alkinyl bedeutet, zu verstehen.

Die erfindungsgemässen Verbindungen sind herbizid wirksam und eignen sich als Wirkstoffe von Unkrautbekämpfungsmitteln. Somit umfasst die Erfindung auch Unkrautbekämpfungsmittel, welche erfindungsgemässe Verbindungen als Wirkstoffe enthalten, Verfahren zur Herstellung dieser Verbindungen sowie die Verwendung der Verbindungen bzw. Mittel zur Bekämpfung von Unkräutern.

In der obigen Formel I umfasst "Halogen" Fluor, Chlor, Brom und Jod. Die Alkyl-, Alkenyl- und Alkinylreste können geradkettig oder verzueigt sein, wobei dies auch für den Alkyl-, Alkenyl- oder Alkinylteil der Halogenalkyl-, Alkylsulfonylalkyl- und anderen Alkyl-, Alkenyl- bzw. Alkinyl-enthaltenden Gruppen gilt. Eine Halogenalkylgruppe kann ein oder mehrere (gleich oder verschiedene) Halogenatome aufweisen.

Beispiele der Reste $R^2$ sind folgende:

Acetylmethyl, 2-Acetyläthyl, Propionylmethyl, Cyanomethyl, Trimethylsilylmethyl, 2-(Trimethylsilyl)-äthyl, 2-(Methylsulfonyl)-äthyl, Methoxycarbonylmethyl, Aethoxycarbonylmethyl, 1-(Methoxycarbonyl)-äthyl, 1-(Aethoxycarbonyl)-äthyl, 1-(Aethoxycarbonyl)-1-methyläthyl, 2-(Aethoxycarbonyl)-1-methyläthyl, 2-Phenoxyäthyl, 2-Cyclohexenyl, 2-Cyanoäthyl, 2-Norbornanyl, 5-Norbornen-2-yl, 2-Allyloxyäthyl und 2-Propargyloxyäthyl.

Bei den Salzen der Verbindungen der Formel I handelt es sich insbesondere um Alkalimetallsalze, z.B. Natrium- und Kaliumsalze; Erdalkalimetallsalze, z.B. Calcium- und Magnesiumsalze; Ammoniumsalze, d.h. unsubstituierte Ammoniumsalze und mono- oder mehrfach-Substituierte Ammoniumsalze, z.B. Triäthylammonium- und Methylammoniumsalze, sowie um Salze mit anderen organischen Basen, z.B. mit Pyridin.

Das Vorhandensein mindestens eines asymmetrischen Kohlenstoffatoms in den Verbindungen der Formel I hat zur Folge, dass die Verbindungen in optisch isomeren Formen auftreten können. Durch das Vorliegen einer allfälligen aliphatischen C=C-Doppelbindung kann auch geometrische Isomerie auftreten. Die Formel I soll all diese möglichen isomeren Formen sowie Gemische davon umfassen.

Bedeutet $R^1$ $C_{3\ \text{oder}\ 4}$-Alkenyl, $C_{3\ \text{oder}\ 4}$-Alkinyl oder $C_{1-4}$-Halogenalkyl, ist dieser Rest vorzugsweise Allyl, Propargyl bzw. Difluormethyl. Falls $R^6$ Halogenalkyl bedeutet, ist dies vorzugsweise Trifluormethyl oder Pentafluoräthyl. Im allgemeinen ist ein allfällig vorkommendes Halogenatom vorzugsweise Fluor, Chlor oder Brom.

Eine interessante Gruppe erfindungsgemässer Verbindungen besteht aus denjenigen Verbindungen der Formel I, in denen $R^2$ verschieden von Carboxy-$C_{1-4}$-alkyl ist und $R^5$ Wasserstoff, Fluor oder $C_{1-4}$-Alkyl und $R^6$ $C_{1-4}$-Alkyl oder $C_{1-4}$-Halogenalkyl bedeuten, sowie deren Enoläthern und Salzen.

Unabhängig voneinander bedeuten $R^1$ vorzugsweise geradkettiges $C_{1-4}$-Alkyl, insbesondere Methyl; $R^2$ vorzugsweise $C_{2-7}$-Alkanoyl-$C_{1-4}$-alkyl, ($C_{1-6}$-Alkoxy)carbonyl-$C_{1-4}$-alkyl, {[$C_{3-9}$-($\alpha$-Alkylalkyliden)]-iminooxy-($C_{1-6}$-alkoxy)carbonyl}-$C_{1-4}$-alkyl, ($C_{3-6}$-Alkenyloxy)carbonyl-$C_{1-4}$-alkyl, ($C_{3-6}$-Alkinyloxy)-carbonyl-$C_{1-4}$-alkyl oder ($C_{3-8}$-Cycloalkyloxy)carbonyl-$C_{1-4}$-alkyl; $R^3$ vorzugsweise Chlor, Brom oder Cyano; $R^4$ vorzugsweise Wasserstoff oder Fluor; $R^5$ vorzugsweise Wasserstoff, Fluor oder Methyl; und $R^6$ vorzugsweise Methyl, Trifluormethyl oder Pentafluoräthyl.

Bevorzugte einzelne Verbindungen der Formel I sind:

2-{2-Chlor-5-[3,6-dihydro-3,4-dimethyl-2,6-dioxo-1(2H)-pyrimidinyl]-4-fluorbenzoyloxy}-2-methyl-propionsäure-äthylester,

2-{2-Chlor-5-[3,6-dihydro-3,4-dimethyl-2,6-dioxo-1(2H)-pyrimidinyl]-4-fluorbenzoyloxy}-2-methyl-propionsäure-methylester,

2-{2-Chlor-5-[3,6-dihydro-3,4-dimethyl-2,6-dioxo-1(2H)-pyrimidinyl]-4-fluorbenzoyloxy}-2-methyl-propionsäure-(tert.butyl)ester,

2-Aethyl-2-{2-chlor-5-[3,6-dihydro-3,4-dimethyl-2,6-dioxo-1(2H)-pyrimidinyl]-4-fluorbenzoyloxy}-propionsäure-äthylester,

2-{2-Chlor-5-[3,6-dihydro-2,6-dioxo-3-methyl-4-trifluormethyl-1(2H)-pyrimidinyl]-benzoyloxy}-2-methyl-propionsäure-methylester,

2-{2-Chlor-5-[3,6-dihydro-2,6-dioxo-3-methyl-4-trifluormethyl-1(2H)-pyrimidinyl]-benzoyloxy}-propionsäure-isopropylester,

2-Aethyl-2-{2-chlor-5-[3,6-dihydro-2,6-dioxo-3-methyl-4-trifluormethyl-1(2H)-pyrimidinyl]-benzoyloxy}-propionsäure-äthylester,

2-{2-Chlor-5-[3,6-dihydro-2,6-dioxo-3-methyl-4-trifluormethyl-1(2H)-pyrimidinyl]-benzoyloxy}-2-methyl-propionsäure-(tert.butyl)ester,

2-{2-Chlor-5-[3,6-dihydro-2,6-dioxo-3-methyl-4-trifluormethyl-1(2H)-pyrimidinyl]-benzoyloxy}-2-methyl-propionsäure-allylester,

2-{2-Chlor-5-[3,6-dihydro-2,6-dioxo-3-methyl-4-trifluormethyl-1(2H)-pyrimidinyl]-benzoyloxy}-2-methyl-propionsäure-cyclopentylester,

2-{2-Chlor-5-[3,6-dihydro-2,6-dioxo-3-methyl-4-trifluormethyl-1(2H)-pyrimidinyl]-benzoyloxy}-propionsäure-äthylester,

2-{2-Chlor-5-[3,6-dihydro-2,6-dioxo-3-methyl-4-trifluormethyl-1(2H)-pyrimidinyl]-benzoyloxy}-2-methyl-propionsäure-äthylester,

2-Chlor-5-[3,6-dihydro-2,6-dioxo-3-methyl-4-trifluormethyl-1(2H)-pyrimidinyl]-benzoesäure-(1-acetyläthyl)ester und

3-{2-Chlor-5-[3,6-dihydro-2,6-dioxo-3-methyl-4-trifluormethyl-1(2H)-pyrimidinyl]-benzoyloxy}-butansäure-äthylester.

Das erfindungsgemässe Verfahren zur Herstellung der Verbindungen der Formel I und ihrer Enoläther sowie Salze ist dadurch gekennzeichnet, dass man

a) zwecks Herstellung derjenigen Verbindungen der Formel I, in denen $R^1$ Wasserstoff bedeutet, sowie gewünschtenfalls von Metallsalzen dieser Verbindungen, eine Verbindung der allgemeinen Formel

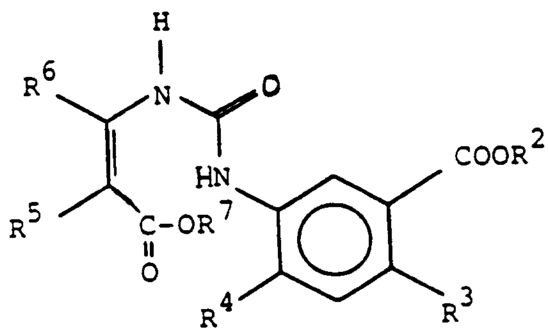

II

worin $R^2$, $R^3$, $R^4$, $R^5$ und $R^6$ die oben angegebenen Bedeutungen besitzen und $R^7$ nieder Alkyl, vorzugsweise $C_{1-4}$-Alkyl, bedeutet,
einer Cyclisierung unter basischen Bedingungen unterwirft und gewünschtenfalls ein allfällig erhaltenes Metallsalz des Uracilderivats der Formel I durch Behandlung mit einer Säure in die saure Form ($R^1$ = Wasserstoff) überführt,
b) zwecks Herstellung derjenigen Verbindungen der Formel I, in denen $R^1$ Wasserstoff bedeutet und $R^6$ verschieden von $C_{1-4}$-Halogenalkyl ist, sowie gewünschtenfalls von Metallsalzen dieser Verbindungen, eine Verbindung der allgemeinen Formel

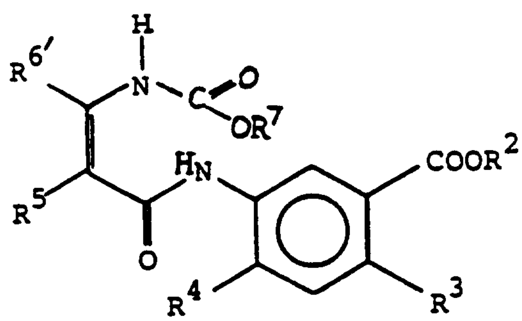

III

worin $R^2$, $R^3$, $R^4$, $R^5$ und $R^7$ die oben angegebenen Bedeutungen besitzen und $R^{6'}$ $C_{1-4}$-Alkyl oder zusammen mit $R^5$ Tri- oder Tetramethylen bedeutet,
einer Cyclisierung unter basischen Bedingungen unterwirft und gewünschtenfalls ein allfällig erhaltenes Metallsalz des Uracilderivats der Formel I durch Behandlung mit einer Säure in die saure Form ($R^1$ = Wasserstoff) überführt,
c) zwecks Herstellung derjenigen Verbindungen der Formel I, in denen $R^1$ $C_{1-4}$-Alkyl, $C_{3\ oder\ 4}$-Alkenyl, $C_{3\ oder\ 4}$-Alkinyl oder $C_{1-4}$-Halogenalkyl bedeutet, ein Uracilderivat der allgemeinen Formel

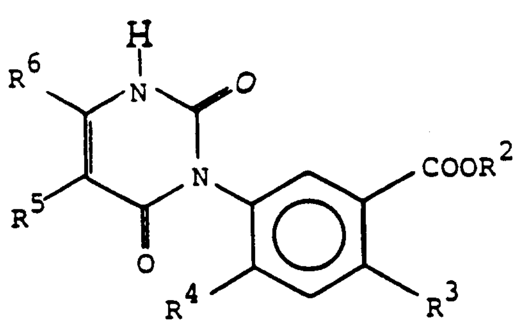

I'

worin $R^2$, $R^3$, $R^4$, $R^5$ und $R^6$ die oben angegebenen Bedeutungen besitzen, einer Alkylierung mit einem entsprechenden, eine $C_{1-4}$-Alkyl-, $C_{3\ oder\ 4}$-Alkenyl-, $C_{3\ oder\ 4}$-Alkinyl- oder $C_{1-4}$-Halogenalkylgruppe enthaltenden Alkylierungsmittel unterwirft,

d) zwecks Herstellung derjenigen Verbindungen der Formel I, in denen $R^1$ verschieden von Wasserstoff ist, und der entsprechenden Enoläther eine Benzoesäure der allgemeinen Formel

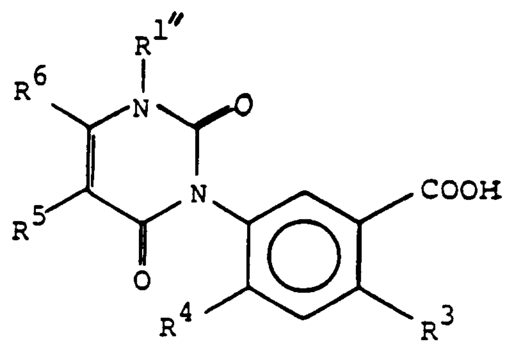

IV

worin $R^{1''}$ $C_{1-4}$-Alkyl, $C_{3\ oder\ 4}$-Alkenyl, $C_{3\ oder\ 4}$-Alkinyl oder $C_{1-4}$-Halogenalkyl bedeutet und $R^3$, $R^4$, $R^5$ und $R^6$ die oben angegebenen Bedeutungen besitzen, oder den entsprechenden Enoläther, wobei die Benzoesäure bzw. deren Enoläther in Form eines reaktionsfähigen Derivats vorliegen kann, mit einer Hydroxyverbindung der allgemeinen Formel

HO-$R^2$     V

worin $R^2$ die oben angegebene Bedeutung besitzt, oder mit einem reaktionsfähigen Derivat dieser Hydroxyverbindung verestert,

e) zwecks Herstellung derjenigen Verbindungen der Formel I, in denen $R^1$ verschieden von Wasserstoff ist, einen Benzoesäureester der allgemeinen Formel

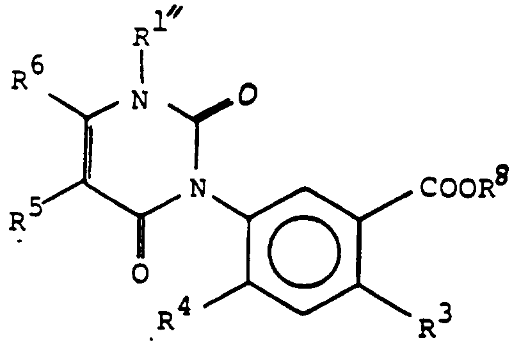

VI

worin $R^{1''}$, $R^3$, $R^4$, $R^5$ und $R^6$ die oben angegebenen Bedeutungen besitzen und $R^8$ $C_{1-6}$-Alkyl, $C_{2-4}$-Alkenyl, $C_{2-4}$-Alkinyl oder $C_{2-6}$-Alkoxyalkyl bedeutet, einer Umesterungsreaktion mit einer Hydroxyverbindung der oben angegebenen Formel V unterwirft, wobei das Reagens V höher siedend ist als das Alkanol, Alkenol bzw. Alkinol $R^8$OH,

f) zwecks Herstellung derjenigen Verbindungen der Formel I, in denen $R^3$ Cyano bedeutet, und der entsprechenden Enoläther einen 2-Halogen-benzoesäureester der allgemeinen Formel

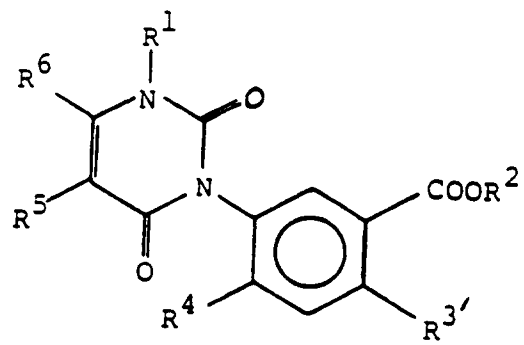

I''

worin $R^{3'}$ Halogen, vorzugsweise Chlor oder Brom, bedeutet und $R^1$, $R^2$, $R^4$, $R^5$ und $R^6$ die oben angegebenen Bedeutungen besitzen,
oder den entsprechenden Enoläther mit einem Metallcyanid behandelt,
g) zwecks Herstellung der Enoläther der Verbindungen der Formel I, ein Uracilderivat der allgemeinen Formel

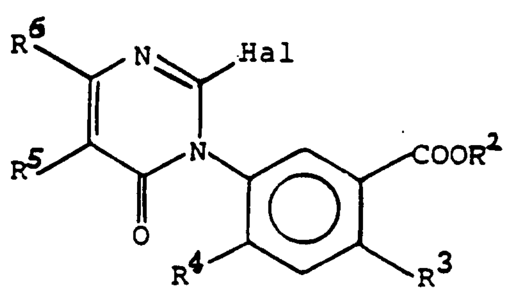

VII

worin $R^2$, $R^3$, $R^4$, $R^5$ und $R^6$ die oben angegebenen Bedeutungen besitzen und Hal Chlor oder Brom bedeutet,
mit einem Alkanol, Alkenol oder Alkinol $R^{1'}OH$ in Gegenwart einer organischen Base oder mit dem entsprechenden Alkoholat, Alkenolat bzw. Alkinolat der allgemeinen Formel

$R^{1'}O^{\ominus}M^{\oplus}$ VIII

worin $R^{1'}$ die oben angegebene Bedeutung besitzt und
$M^{\oplus}$ ein Aequivalent eines Metallions bedeutet,
behandelt,
und gewünschtenfalls eine so erhaltene Verbindung der Formel I, in der $R^1$ Wasserstoff bedeutet, in ein Salz überführt.

Die Cyclisierung nach Verfahrensvariante a) oder b) kann zweckmässigerweise durchgeführt werden, indem man die Verbindung der Formel II bzw. III in einem inerten protischen organischen Lösungsmittel, wie einem Alkohol, z.B. Methanol, Aethanol oder Isopropanol; einem inerten aprotischen organischen Lösungsmittel, wie einem aliphatischen oder cyclischen Aether, z.B. 1,2-Dimethoxyäthan, Tetrahydrofuran oder Dioxan, oder einem Aromaten, z.B. Benzol oder Toluol; einem inerten aprotischen, polaren organischen Lösungsmittel, z.B. Dimethylformamid oder Dimethylsulfoxid, wobei solche Lösungsmittel gegebenenfalls im Zweiphasen-Gemisch mit einem Kohlenwasserstoff, z.B. n-Hexan oder Toluol, verwendet werden können; oder Wasser mit einer Base bei Temperaturen zwischen -78 °C und der Rückflusstemperatur des Reaktionsgemisches behandelt. Als Basen kommen vorzugsweise Natriumalkoholate, Alkalimetallhydroxide, insbesondere Natriumhydroxid und Kaliumhydroxid, Alkalimetallcarbonate, insbesondere Natriumcarbonat und Kaliumcarbonat, und Natriumhydrid in Betracht. Falls als Lösungsmittel ein Alkanol verwendet wird, entspricht dieses Lösungsmittel zweckmässigerweise der jeweiligen Hydroxyverbindung $R^2$-OH; dadurch werden unerwünschte konkurrierende Umesterungsreaktionen vermieden. Bei der Verwendung von Natriumhydrid als Base ist das Lösungsmittel vorzugsweise ein aliphatischer oder cyclischer Aether, Dimethylformamid oder Dimethylsulfoxid, wobei jedes dieser Lösungsmittel im Gemisch mit Toluol verwendet werden kann.

Nach Beendigung der Cyclisierung liegt das Produkt im Falle der Verwendung einer der obenerwähnten Basen oder dergleichen in Form des entsprechenden Alkalimetallsalzes vor. Dieses kann in an sich bekannter Weise isoliert und gereinigt werden, oder man kann das Gemisch ansäuern, um die jeweilige Verbindung der Formel I an sich zu isolieren. Zu diesem Zwecke verwendet man vorzugsweise eine Mineralsäure, wie Salzsäure, oder eine starke organische Säure, wie Essigsäure oder p-Toluolsulfonsaure.

Bei der Verfahrensvariante c) steht der Ausdruck "Alkylierung" für die Substitution des Wasserstoffatoms des $N^1$-Atoms des Uracilkerns mit einer $C_{1-4}$-Alkyl-, $C_{3\ \mathrm{oder}\ 4}$-Alkenyl, $C_{3\ \mathrm{oder}\ 4}$-Alkinyl- oder $C_{1-4}$-Halogenalkylgruppe. Als Alkylierungsmittel wird zweckmässigerweise ein $C_{1-4}$-Alkyl, $C_{3\ \mathrm{oder}\ 4}$-Alkenyl- oder $C_{3\ \mathrm{oder}\ 4}$-Alkinylhalogenid, insbesondere das diesbezügliche Chlorid oder Bromid, oder -sulfat bzw. ein mehrfach halogeniertes $C_{1-4}$-Alkan, wie beispielsweise Chlordifluormethan, oder ein mono- oder mehrfach halogeniertes Alken, wie beispielsweise Tetrafluoräthen, verwendet.

Die Alkylierung wird zweckmässigerweise in Gegenwart eines inerten, protischen organischen Lösungsmittels, wie eines niederen Alkanols, z.B. Aethanol, gegebenenfalls im Gemisch mit Wasser; eines inerten, aprotischen organischen Lösungsmittels, wie eines aliphatischen oder cyclischen Aethers, z.B. 1,2-Dimethoxyäthan, Tetrahydrofuran oder Dioxan; eines Ketons, z.B. Aceton oder Butan-2-on; oder eines inerten, aprotischen, polaren organischen Lösungsmittels, z.B. Dimethylformamid, Dimethylsulfoxid oder Acetonitril, sowie in Gegenwart einer Base, wie Natriumhydrid, eines Alkalimetallhydroxids, insbesondere Natrium- oder Kaliumhydroxid, eines Alkalimetallalkoholats, insbesondere Natriumalkoholat, oder eines Alkalimetallcarbonats oder -hydrogencarbonats, insbesondere Natriumcarbonat, Kaliumcarbonat, Natriumhydrogencarbonat oder Kaliumhydrogencarbonat, bei Temperaturen zwischen 0 °C und der Rückflusstemperatur des Reaktionsgemisches, vorzugsweise bei Raumtemperatur oder, im Falle der Substitution des Wasserstoffatoms des $N^1$-Atoms mit einer $C_{1-4}$-Halogenalkylgruppe, vorzugsweise bei Temperaturen zwischen 50 °C und 100 °C, durchgeführt. In einer bevorzugten Ausführungsform wird das Uracilderivat der Formel I' zunächst mit der Base, wie Natriumhydrid, -äthanolat oder -carbonat, im Lösungsmittel behandelt und nach einer kurzen Reaktionszeit mit dem Halogenid im gleichen Lösungsmittel versetzt. In einer weiteren Ausführungsform wird das Uracilderivat I' zusammen mit einem Dialkylsulfat in Gegenwart eines Alkalimetallhydrogencarbonats, insbesondere Natrium- oder Kaliumhydrogencarbonat, im Lösungsmittel, z.B. Aceton, bei Rückflusstemperatur zur Reaktion gebracht. Die Reaktion ist in der Regel je nach verwendetem Lösungsmittel innert relativ kurzer Zeit oder nach wenigen Stunden beendet.

Bei der Verfahrensvariante d) handelt es sich um eine Veresterung der Benzoesäure oder des Enoläthers bzw. eines reaktionsfähigen Derivats davon, die nach an sich bekannten Methoden durchgeführt werden kann. So wird beispielsweise ein Salz einer Benzoesäure der Formel IV oder des entsprechenden Enoläthers mit einem Halogenid, insbesondere Chlorid, Bromid oder Jodid, oder dem Sulfat, Mesylat oder Tosylat der Hydroxyverbindung V in einem inerten Verdünnungsmittel bei Temperaturen zwischen der Raumtemperatur und 100 °C, z.B. bei der Rückflusstemperatur des Reaktionsgemisches, vorzugsweise im Temperaturbereich von 40 °C bis 70 °C, umgesetzt. Es kommen als Salze der Benzoesäure der Formel IV oder des entsprechenden Enoläthers insbesondere Alkalimetallsalze, z.B. das Natrium-, Kalium- oder Lithiumsalz, Erdalkalimetallsalze, z.B. das Magnesium, Calcium- oder Bariumsalz, und Salze mit organischen Basen, wie tertiäre Amine, z.B. Triäthylamin, 1,5-Diaza-bicyclo[4,3,0]non-5-en, 1,8-Diaza-bicyclo-[5,4,0]undec-7-en und 1,4-Diaza-bicyclo[2,2,2]octan, in Frage, wobei die Alkalimetallsalze, insbesondere das Natriumsalz und das Kaliumsalz, bevorzugt sind. Die verwendbaren Verdünnungsmittel sind vorzugsweise inerte organische Lösungsmittel, wie niedere Alkanole, z.B. Aethanol, aliphatische und cyclische Aether, z.B. Diäthyläther, Tetrahydrofuran und Dioxan, Ketone, z.B. Aceton und 2-Butanon, Dimethylformamid, Dimethylsulfoxid, Acetonitril und Hexamethylphosphorsäuretriamid. Das Salz kann in situ hergestellt werden, indem man die Säure mit einer geeigneten anorganischen Base, z.B. einem Alkalimetall- oder Erdalkalimetallcarbonat, -hydrogencarbonat, -hydroxid oder -hydrid, bzw. organischen Base, zum Salz umsetzt und dies anschliessend im gleichen Reaktionsmedium mit dem zweiten Reaktionspartner reagieren lässt.

Im Falle der Verwendung eines Säurehalogenids der Benzoesäure der Formel IV oder des entsprechenden Enoläthers als reaktionsfähiges Derivat wird dies zweckmässigerweise mit der Hydroxyverbindung der Formel V in einem inerten organischen Lösungsmittel, wie einem aliphatischen oder cyclischen Aether, z.B. Diäthyläther, 1,2-Dimethoxyäthan, Tetrahydrofuran oder Dioxan, einem aliphatischen oder aromatischen Kohlenwasserstoff, z.B. n-Hexan, Benzol oder Toluol, oder einem halogenierten, insbesondere chlorierten, Kohlenwasserstoff, z.B. Methylenchlorid, Chloroform oder Tetrachlorkohlenstoff, bei Temperaturen von etwa -20 °C bis 100 °C, vorzugsweise von 0 °C bis 50 °C, umgesetzt. Zudem wird zweckmässigerweise in Gegenwart eines säurebindenden Mittels gearbeitet, wie einer organischen Base, z.B. Triäthylamin, Pyridin,

4-Dimethylaminopyridin, 1,5-Diaza-bicyclo[4,3,0]non-5-en, 1,8-Diaza-bicyclo[5,4,0]undec-7-en oder 1,4-Diaza-bicyclo[2,2,2]octan. Das Säurehalogenid ist vorzugsweise das Säurechlorid.

Als weitere in Frage kommende reaktionsfähige Derivate der Benzoesäure der Formel IV oder des entsprechenden Enoläthers seien der entsprechende O-Acyl-1,3-dicyclohexylisoharnstoff und das entsprechende N-Acylimidazol oder Säureanhydrid genannt. Solche Derivate können wie das Säurehalogenid mit den Hydroxyverbindungen der Formel V umgesetzt werden, um zu den gewünschten Benzoesäureestern zu gelangen. In diesen Fällen erübrigt sich jedoch die Verwendung eines säurebindenden Mittels.

Die Umsetzung nach Verfahrensvariante e) kann zweckmässigerweise durchgeführt werden, indem man den Benzoesäureester der Formel VI in überschüssiger Hydroxyverbindung der Formel V in Gegenwart eines basischen Katalysators, wie Natriumcyanid, erhitzt, und zwar vorzugsweise bei der Rückflusstemperatur des Reaktionsgemisches. Im Laufe der Reaktion wird der Rest $R^8$ des Benzoesäureesters VI durch die Gruppe $R^2$ der Hydroxyverbindung V ersetzt, wobei das niedriger siedende Alkanol, Alkenol bzw. Alkinol $R^8OH$ freigesetzt wird.

Bei der Verfahrensvariante f) handelt es sich um eine Austauschreaktion das Halogensubstituenten des Benzolkerns. Dieses Halogenatom wird also durch die Cyanogruppe ersetzt, und zwar mittels des Metallcyanids. Letzteres ist insbesondere ein Uebergangsmetallcyanid, vorzugsweise Kupfer(I)cyanid. Die Umsetzung erfolgt zweckmässigerweise in Gegenwart eines aprotischen, polaren Lösungsmittels, wie eines Alkylnitrils, z.B. Aceto-, Propio- oder Butyronitril; eines Alkylharnstoffes, z.B. N,N,N',N'-Tetramethylharnstoff; eines Dialkylamids, z.B. Dimethylformamid; eines Dialkylsulfoxids, z.B. Dimethylsulfoxid; N-Methyl-2-pyrrolidon; 1,3-Dimethyl-imidazolidin-2-on; 1,3-Dimethyl-3,4,5,6-tetrahydro-2(1H)-pyrimidinon; oder Hexamethylphosphorsäuretriamid, bei erhöhten Temperaturen, und zwar zwischen 150 °C und 250 °C. Im Ausgangsmaterial I'' bedeutet $R^4$ vorzugsweise Wasserstoff oder Fluor.

Bei der Verfahrensvariante g) steht der Ausdruck "Metallion" insbesondere für ein Alkalimetallion, z.B. das Natrium- oder Kaliumion, oder ein Erdalkalimetallion, z.B. das Calcium oder Magnesiumion. Das Natriumion ist das bevorzugte Metallion. Im Falle der Verwendung des Alkanols, Alkenols oder Alkinols $R^{1'}OH$ ist insbesondere Pyridin die geeignete organische Base.

Die Umsetzung erfolgt zweckmässigerweise in einem Ueberschuss an dem entsprechenden Alkohol $R^{1'}OH$ als Verdünnungsmittel und bei Temperaturen zwischen 0 °C und 50 °C, vorzugsweise bei Raumtemperatur.

Sofern sie nicht unmittelbar durch die oben beschriebene unter basischen Bedingungen durchgeführte Cyclisierung herstellbar sind, können die gewünschten Salze der Verbindungen der Formel I, in denen $R^1$ Wasserstoff bedeutet, auch aus diesen Verbindungen I in an sich bekannter Weise hergestellt werden, wie beispielsweise durch Auflösen der Verbindung der Formel I in einer Lösung einer diesbezüglichen anorganischen oder organischen Base. Die Salzbildung erfolgt in der Regel innert kurzer Zeit bei Raumtemperatur. In einer Ausführungsform wird das Natriumsalz durch Auflösen des Uracilderivats I in wässriger Natriumhydroxidlösung bei Raumtemperatur hergestellt, wobei äquivalente Mengen des Uracilderivats und des Natriumhydroxids verwendet werden. Das feste Salz kann dann durch Fällen mit einem geeigneten inerten Lösungsmittel oder durch Abdampfen des Lösungsmittels isoliert werden. Eine weitere Ausführungsform besteht darin, eine wässrige Lösung eines Alkalimetallsalzes des Uracilderivats I in eine wässrige Lösung eines Salzes, das ein anderes Metallion als ein Alkalimetallion aufweist, einzuführen, wobei das zweite Metallsalz des Uracilderivats hergestellt wird. Diese Ausführungsform dient im allgemeinen zur Herstellung von Uracil-Metallsalzen, die in Wasser unlöslich sind.

Die erhaltenen Verbindungen der Formel I, Enoläther sowie Salze können nach an sich bekannten Methoden isoliert und gereinigt werden. Ferner ist dem Fachmann geläufig, in welcher Reihenfolge allfällige Kombinationen der Verfahrensvarianten c) bis f) zweckmässig durchzuführen sind, um mögliche, unerwünschte konkurrierende Reaktionen zu vermeiden.

Sofern keine gezielte Synthese zur Isolierung reiner Isomerer durchgeführt wird, kann das Produkt als Gemisch zweier oder mehrerer Isomerer anfallen. Die Isomeren können nach an sich bekannten Methoden aufgetrennt werden. Gewünschtenfalls können beispielsweise reine optisch aktive Isomere auch durch Synthese aus entsprechenden optisch aktiven Ausgangsmaterialien hergestellt werden.

Die Ausgangsmaterialien der Formel II, die neu sind, können in an sich bekannter Weise hergestellt werden, z.B. gemäss den nachfolgenden Reaktionsschemata 1 [Methoden aa), bb) und cc)]:

## Reaktionsschemata 1

aa)

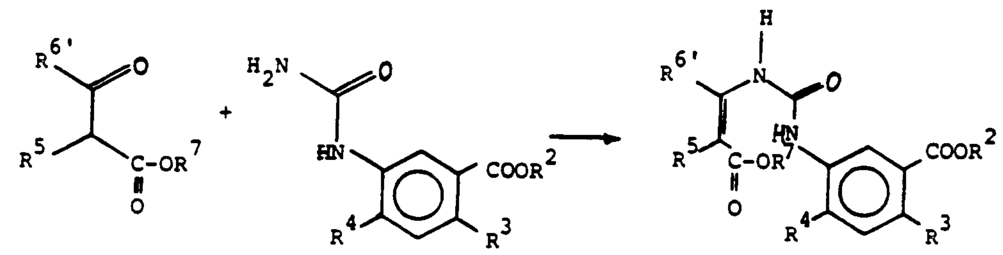

IX X II′

bb)

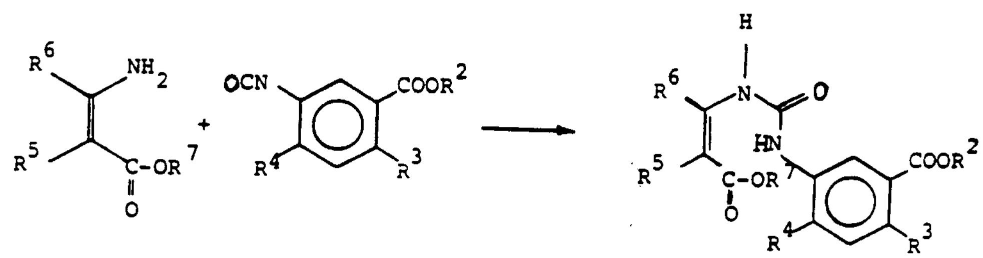

XI XII II

cc)

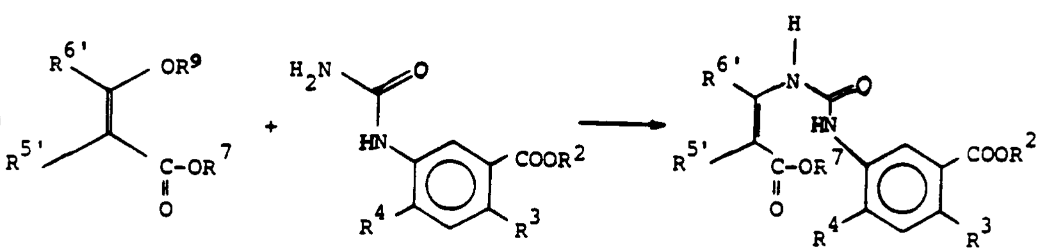

XIII X II″

In den obigen Reaktionsschemata besitzen $R^2$, $R^3$, $R^4$, $R^5$, $R^6$, $R^{6'}$ und $R^7$ die oben angegebenen Bedeutungen; $R^{5'}$ bedeutet Wasserstoff, $C_{1-4}$-Alkyl oder zusammen mit $R^{6'}$ Tri- oder Tetramethylen; und $R^9$ bedeutet nieder Alkyl, vorzugsweise $C_{1-4}$-Alkyl.

Die Methode aa) wird zweckmässigerweise dadurch durchgeführt, dass man die Verbindungen der Formeln IX und X in einem im wesentlichen wasserfreien Verdünnungsmittel und in Gegenwart eines sauren Katalysators bei erhöhter Temperatur miteinander reagieren lässt. Als Verdünnungsmittel kommen insbesondere mit Wasser Azeotrope bildende organische Lösungsmittel, wie Aromaten, z.B. Benzol, Toluol und Xylole; halogenierte Kohlenwasserstoffe, wie Methylenchlorid, Chloroform, Tetrachlorkohlenstoff und Chlorbenzol; und aliphatische und cyclische Aether, wie 1,2-Dimethoxyäthan, Tetrahydrofuran und Dioxan, und als saure Katalysatoren insbesondere starke Mineralsäuren, wie Schwefelsäure und Salzsäure; organische

Säuren, wie p-Toluolsulfonsäure; Phosphor enthaltende Säuren, wie Orthophosphorsäure und Polyphosphorsäure; und saure Kationenaustauscher, wie "Amberlyst 15" (Fluka), in Frage. Man arbeitet im allgemeinen in einem Temperaturbereich von etwa 70°C bis 120°C, vorzugsweise bei der Rückflusstemperatur des Reaktionsgemisches. Unter diesen Reaktionsbedingungen wird die erwünschte rasche Entfernung des in der Reaktion gebildeten Wassers erzielt.

Die Umsetzung nach der Methode bb) erfolgt zweckmässigerweise in Gegenwart eines im wesentlichen wasserfreien aprotischen organischen Lösungsmittels, wie eines aliphatischen oder cyclischen Aethers, z.B. Diäthyläther, 1,2-Dimethoxyäthan, Tetrahydrofuran oder Dioxan, eines aliphatischen oder aromatischen Kohlenwasserstoffs, z.B. n-Hexan, Benzol, Toluol oder eines Xylols; oder eines halogenierten, aliphatischen Kohlenwasserstoffs, z.B. Methylenchlorid, Chloroform, Tetrachlorkohlenstoff oder 1,2-Dichloräthan, sowie gegebenenfalls in Gegenwart einer Base, insbesondere einer organischen tertiären Base, wie Triäthylamin oder Pyridin, wobei letzteres sowohl als Lösungsmittel wie auch als Base dienen kann, oder eines Metallhydrids, wie Natrium- oder Kaliumhydrid. Die Reaktionstemperaturen sind vorzugsweise im Bereich von etwa -80°C bis 50°C, wobei besonders bevorzugt bei Temperaturen zwischen -30°C und der Raumtemperatur gearbeitet wird.

Die Umsetzung nach der Methode cc) wird zweckmässigerweise in einem inerten, mit Wasser mischbaren, organischen Lösungsmittel, wie einem aliphatischen oder cyclischen Aether, z.B. 1,2-Dimethoxyäthan, Tetrahydrofuran oder Dioxan, oder einem niederen Alkanol, wie Aethanol, bei Temperaturen zwischen 50°C und 100°C, vorzugsweise bei der Rückflusstemperatur des Reaktionsgemisches, oder in einem aromatischen Lösungsmittel, wie Benzol, Toluol oder einem Xylol in Gegenwart eines sauren Katalysators, wie Salzsäure oder p-Toluolsulfonsäure, bei Temperaturen zwischen 50°C und 100°C, vorzugsweise 60°C bis 80°C, durchgeführt.

Die Ausgangsmaterialien der Formel III sind ebenfalls neu und können in an sich bekannter Weise hergestellt werden, z.B. gemäss den nachfolgenden Reaktionsschemata 2 [Methoden dd), ee) und ff)]:

## Reaktionsschemata 2

dd)

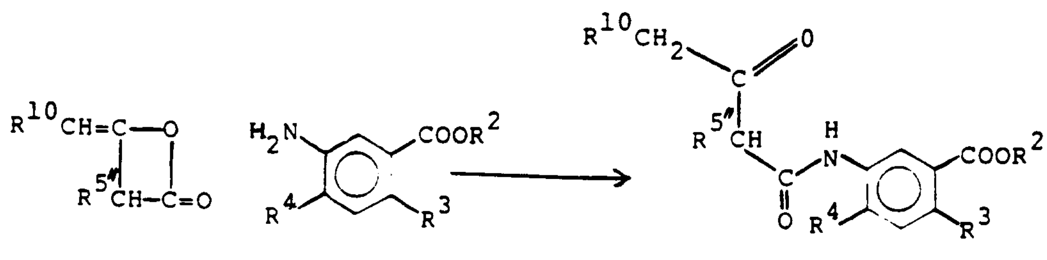

XIV XV XVI

ee)

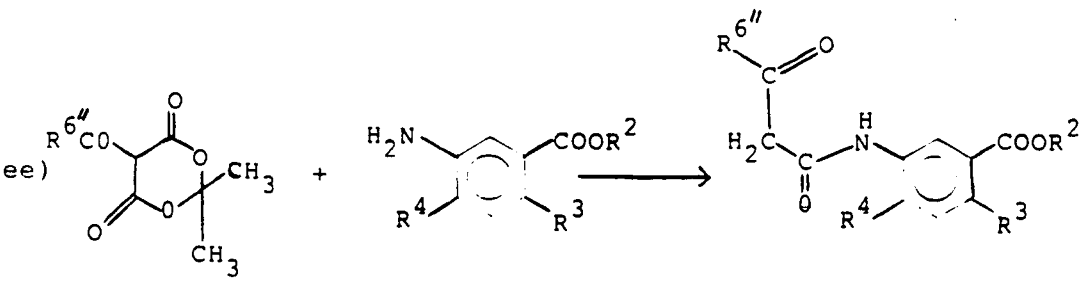

XVII XV XVIII

ff)

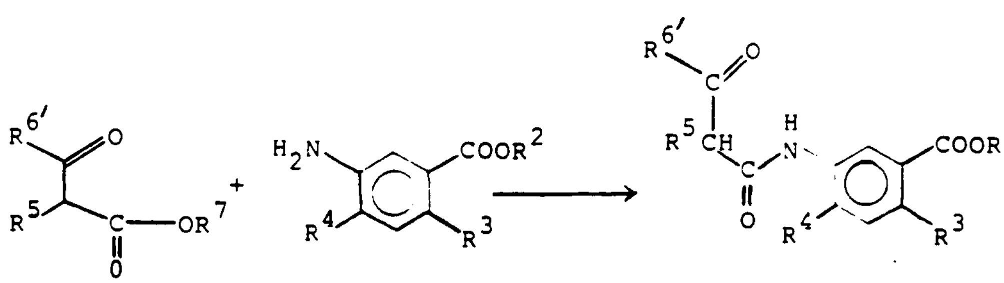

IX XV XIX

XVI/XVIII/XIX + $H_2NCOOR^7$ ⟶ III

XX

In den obigen Reaktionsschemata besitzen $R^2$, $R^3$, $R^4$, $R^5$, $R^{6'}$ und $R^7$ die oben angegebenen Bedeutungen; $R^{5''}$ bedeutet Wasserstoff oder $C_{1-4}$-Alkyl; $R^{6''}$ bedeutet $C_{1-4}$-Alkyl; und $R^{10}$ bedeutet Wasserstoff oder $C_{1-3}$-Alkyl.

Die Umsetzung des Amins der Formel XV mit dem Diketen der Formel XIV nach der Methode dd) erfolgt zweckmässigerweise in einem wasserfreien inerten aprotischen Lösungsmittel, wie einem halogenierten Kohlenwasserstoff, z.B. Methylenchlorid, Chloroform, Tetrachlorkohlenstoff oder Chlorbenzol, einem aromatischen Kohlenwasserstoff, z.B. Benzol, Toluol oder einem Xylol, oder einem aliphatischen oder cyclischen Aether, z.B. Diäthyläther, 1,2-Dimethoxyäthan, Tetrahydrofuran oder Dioxan, in Gegenwart eines basischen Katalysators, wie 4-Pyrrolidino-pyridin, 4-Dimethylaminopyridin, 1,4-Diazabicyclo[2,2,2]octan, 1,5-Diazabicyclo[4,3,0]non-5-en, 1,8-Diazabicyclo[5,4,0]undec-7-en oder Diäthylamin. Da die Reaktion exothermisch ist, arbeitet man im allgemeinen in einem Temperaturbereich von -10 °C bis 50 °C, vorzugsweise bei Raumtemperatur.

Die Umsetzung der Verbindungen der Formeln XVII und XV miteinander nach der Methode ee) wird zweckmässigerweise in einem wasserfreien, inerten, aprotischen Lösungsmittel bei Temperaturen von etwa 70 °C bis 140 °C, vorzugsweise von 100 °C bis 120 °C, durchgeführt. Als derartige Lösungsmittel eignen sich insbesondere Aromaten, z.B. Benzol, Toluol und Xylole; halogenierte Kohlenwasserstoffe, z.B. Tetrachlorkohlenstoff, Trichloräthan, Tetrachloräthan und Chlorbenzol; und aliphatische und cyclische Aether, z.B. Dibutyläther, 1,2-Dimethoxyäthan, Tetrahydrofuran und Dioxan.

Bei der Umsetzung nach der Methode ff) handelt es sich um eine Aminolyse, die zweckmässigerweise in einem wasserfreien Lösungsmittel, oder ohne Lösungsmittel [siehe beispielsweise J.Soc.Dyes Col. 42, 81 (1926), Ber. 64, 970 (1931) und J.A.C.S. 70, 2402 (1948)] bei erhöhter Temperatur erfolgt. Als Lösungsmittel kommen insbesondere inerte, aprotische Lösungsmittel, wie gegebenenfalls halogenierte Aromaten, z.B. Toluol, Xylole und Chlorbenzole, in Frage. Man arbeitet im allgemeinen in einem Temperaturbereich von etwa 130 °C bis 160 °C. Gegebenenfalls wird zudem in Gegenwart eines basischen Katalysators gearbeitet, z.B. eines höher siedenden Amins [siehe beispielsweise Helv. Chim. Acta 11, 779 (1928) und US-Patentschrift Nr. 2.416.738] oder Pyridin.

Die anschliessende Umsetzung der so hergestellten Verbindung der Formel XVI, XVIII bzw. XIX mit dem Carbaminsäure-niederalkylester der Formel XX erfolgt zweckmässigerweise in einem im wesentlichen wasserfreien Verdünnungsmittel und in Gegenwart eines sauren Katalysators bei erhöhter Temperatur. Als Verdünnungsmittel kommen insbesondere mit Wasser Azeotrope bildende organische Lösungsmittel, wie Aromaten, z.B. Benzol, Toluol und Xylole; und halogenierte Kohlenwasserstoffe, wie Tetrachlorkohlenstoff und Chlorbenzol, und als saure Katalysatoren insbesondere starke Mineralsäuren, wie Schwefelsäure; organische Säuren, wie p-Toluolsulfonsäure; Phosphor enthaltende Säuren, wie Orthophosphorsäure und Polyphosphorsäure; und saure Kationenaustauscher, wie "Amberlyst 15" (Fluka), in Frage. Man arbeitet im allgemeinen in einem Temperaturbereich von etwa 70 °C bis 150 °C, vorzugsweise bei der Rückflusstemperatur des Reaktionsgemisches. Unter diesen Reaktionsbedingungen wird die erwünschte rasche Entfernung des in der Reaktion gebildeten Wassers erzielt.

Die Ausgangsmaterialien der Formeln IV und VI und deren Herstellung sind grösstenteils in der europäischen Patentpublikation Nr. 195.346 beschrieben. Diejenigen Ausgangsmaterialien IV und VI, deren Herstellung nicht beschrieben ist, können analog den bekannten Ausgangsmaterialien hergestellt werden. Die ebenfalls als Ausgangsmaterialien verwendbaren reaktionsfähigen Derivate der Benzoesäuren der Formel IV können aus diesen Benzoesäuren nach an sich bekannten Methoden hergestellt werden. Die ebenfalls bei der Verfahrensvariante d) als Ausgangsmaterialien verwendbaren Enoläther der Benzoesäuren IV, d.h. die Verbindungen der allgemeinen Formel

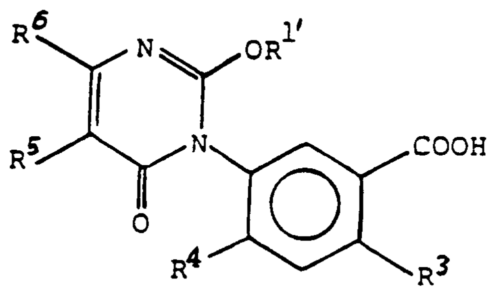

IVa

sind in der internationalen (PCT-) Patentpublikation Nr. WO 88/10254 beschrieben.

Die in der Verfahrensvariante g) verwendeten Ausgangsmaterialien der Formel VII können durch Halogenierung der entsprechenden Uracilderivate der oben angegebenen Formel I' hergestellt werden. Bei dieser Halogenierung wird als Halogenierungsmittel insbesondere Thionylchlorid, Phosphorpentachlorid oder Phosphoroxychlorid bzw. Phosphorpentabromid oder Phosphorylbromid verwendet. Gegebenenfalls wird ein Gemisch von Phosphorpentachlorid und Phosphoroxychlorid bzw. von Phosphorpentabromid und Phosphorylbromid eingesetzt, wobei ein Ueberschuss an Phosphoroxychlorid bzw. Phosphorylbromid als Verdünnungsmittel dienen kann. Die Chlorierung bzw. Bromierung kann in Gegenwart eines inerten Verdünnungsmittels, insbesondere eines aprotischen organischen Lösungsmittels, wie eines aliphatischen oder aromatischen Kohlenwasserstoffes, z.B. n-Hexan, Benzol, Toluol oder eines Xylols; eines halogenierten

aliphatischen Kohlenwasserstoffes, z.B. Methylenchlorid, Chloroform oder 1,2-Dichloräthan; eines halogenierten aromatischen Kohlenwasserstoffes, z.B. Chlorbenzol, oder eines tertiären Amins, z.B. N,N-Dimethylanilin, durchgeführt werden, jedoch ist dies im Falle der Verwendung von Phosphoroxychlorid bzw. Phosphorylbromid als Halogenierungsmittel nicht notwendig. Bei Verwendung von Thionylchlorid als Halogenierungsmittel erweist es sich als zweckmässig, eine katalytische Menge Dimethylformamid zuzusetzen. Die Reaktionstemperaturen liegen im allgemeinen zwischen 0 °C und der Rückflusstemperatur des Reaktionsgemisches, vorzugsweise zwischen 80 °C und 120 °C.

Bei den als Ausgangsmaterialien in den Verfahrensvarianten c) und f) dienenden Uracilderivaten der Formel I' bzw. I'' handelt es sich um Untergruppen von Verbindungen der Formel I. Die restlichen in den Verfahrensvarianten d), f) und g) sowie in den Reaktionsschemata 1 und 2 involvierten Ausgangsmaterialien bzw. Reagenzien sind entweder bekannt oder können nach an sich bekannten Methoden hergestellt werden.

Die Verbindungen der Formel I sowie ihre Enoläther oder Salze sind geeignet, Pflanzenwachstum, insbesondere unerwünschtes Pflanzenwachstum, zu steuern, zu verhindern oder zu zerstören. Die Verbindungen besitzen insbesondere herbizide Eigenschafen und eignen sich zur Bekämpfung von Unkräutern einschliesslich Ungräsern, beispielsweise Abutilon theophrasti, Amaranthus retroflexus, Cassia obtusifolia, Chenopodium album, Chrysanthemum segetum, Datura stramonium, Digitaria sanguinalis, Echinochloa crus-galli, Galium aparine, Matricaria chamomilla, Setaria faberii, Sinapis arvensis und Xanthium pennsylvanicum, in diversen Nutzpflanzenkulturen, beispielsweise Raps-, Soya-, Baumwolle-, Reis-, Weizen- und Maiskulturen. Zudem sind die Verbindungen sowohl Vorauflauf- als auch Nachauflauf-Herbizide. Des weiteren können die Verbindungen zur Kontrolle von unerwünschtem Pflanzenwachstum, z.B. in Kartoffeln, Baumwolle, Sonnenblumen und Wasserunkräutern, verwendet werden. Sie können beispielsweise als Abbrennmittel zur Erleichterung der Ernte bei Kartoffeln und Baumwolle verwendet werden.

In der Praxis genügt üblicherweise eine Konzentration von 1 g bis 3 kg erfindungsgemässe Verbindung/ha, vorzugsweise 10 g bis 1 kg erfindungsgemässe Verbindung/ha, um den gewünschten herbiziden Effekt zu erzielen. Besonders bevorzugt ist die Konzentrationsreihe 15 bis 500 g erfindungsgemässe Verbindung/ha.

Das erfindungsgemässe Unkrautbekämpfungsmittel ist dadurch gekennzeichnet, dass es eine wirksame Menge mindestens einer Verbindung der Formel I, wie oben definiert, oder eines Enoläthers oder Salzes davon sowie Formulierungshilfsstoffe enthält. Das Mittel enthält zweckmässigerweise zumindest einen der folgenden Formulierungshilfsstoffe: feste Trägerstoffe; Lösungs- bzw. Dispersionsmittel; Tenside (Netzmittel und Emulgatoren); Dispergiermittel (ohne Tensidwirkung); und Stabilisatoren. Unter Verwendung solcher und anderer Hilfsstoffe können diese Verbindungen, also die herbiziden Wirkstoffe, in die üblichen Formulierungen übergeführt werden, wie Stäube, Pulver, Granulate, Lösungen, Emulsionen, Suspensionen, emulgierbare Konzentrate, Pasten und dergleichen.

Die Verbindungen der Formel I und ihre Enoläther sind im allgemeinen wasserunlöslich, die Salze hingegen, insbesondere die Alkalimetallsalze und Ammoniumsalze, im allgemeinen wasserlöslich, und können nach den für wasserunlösliche bzw. wasserlösliche Verbindungen üblichen Methoden unter Verwendung der diesbezüglichen Formulierungshilfsstoffe konfektioniert werden. Die Herstellung der Mittel kann in an sich bekannter Weise durchgeführt werden, z.B. durch Vermischen des jeweiligen Wirkstoffes mit festen Trägerstoffen, durch Auflösen oder Suspendieren in geeigneten Lösungs- bzw. Dispersionsmitteln, eventuell unter Verwendung von Tensiden als Netzmitteln oder Emulgatoren und/oder von Dispergiermitteln, durch Verdünnen bereits vorbereiteter emulgierbarer Konzentrate mit Lösungs- bzw. Dispersionsmitteln usw.

Als feste Trägerstoffe kommen im wesentlichen in Frage: natürliche Mineralstoffe, wie Kreide, Dolomit, Kalkstein, Tonerden und Kieselsäure und deren Salze (beispielsweise Kieselgur, Kaolin, Bentonit, Talkum, Attapulgit und Montmorrillonit); synthetische Mineralstoffe, wie hochdisperse Kieselsäure, Aluminiumoxid und Silikate; organische Stoffe, wie Cellulose, Stärke, Harnstoff und Kunstharze; und Düngemittel, wie Phosphate und Nitrate, wobei solche Trägerstoffe z.B. als Pulver oder als Granulate vorliegen können.

Als Lösungs- bzw. Dispersionsmittel kommen im wesentlichen in Frage: Aromaten, wie Benzol, Toluol, Xylole und Alkylnaphthaline; chlorierte Aromaten und chlorierte aliphatische Kohlenwasserstoffe, wie Chlorbenzole, Chloräthylene und Methylenchlorid; aliphatische Kohlenwasserstoffe, wie Cyclohexan und Paraffine, z.B. Erdölfraktionen; Alkohole, wie Butanol und Glykol, sowie deren Aether und Ester; Ketone, wie Aceton, Methyläthylketon, Methylisobutylketon und Cyclohexanon; und stark polare Lösungs- bzw. Dispersionsmittel, wie Dimethylformamid, N-Methylpyrrolidon und Dimethylsulfoxid, wobei solche Lösungsmittel vorzugsweise Flammpunkte von mindestens 30 °C und Siedepunkte von mindestens 50 °C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol-Treibgase, wie Kohlenwasserstoffe, z.B. Propan und Isobutan, und Halogenkohlenwasserstoffe, z.B. Dichlordifluormethan. Liegt das erfindungsgemässe Unkrautbekämpfungsmittel in Form einer Druckgaspackung vor, so wird zweckmässigerweise zusätzlich zum Treibgas ein Lösungsmittel verwendet.

Die Tenside (Netzmittel und Emulgatoren) können nicht-ionische Verbindungen sein, wie Kondensationsprodukte von Fettsäuren, Fettalkoholen oder fettsubstituierten Phenolen mit Aethylenoxid; Fettsäureester und -äther von Zuckern oder mehrwertigen Alkoholen; die Produkte, die aus Zuckern oder mehrwertigen Alkoholen durch Kondensation mit Aethylenoxid erhalten werden; Blockpolymere von Aethylenoxid und Propylenoxid; oder Alkyldimethylaminoxide.

Die Tenside können auch anionische Verbindungen sein, wie Seifen; Fettsulfatester, z.B. Dodecylnatriumsulfat, Octadecylnatriumsulfat und Cetylnatriumsulfat; Alkylsulfonate, Arylsulfonate und fettaromatische Sulfonate, wie Alkylbenzolsulfonate, z.B. Calcium-dodecylbenzolsulfonat, und Butylnaphthalinsulfonate; und komplexere Fettsulfonate, z.B. die Amidkondensationsprodukte von Oelsäure und N-Methyltaurin und das Natriumsulfonat von Dioctylsuccinat.

Die Tenside können schliesslich kationische Verbindungen sein, wie Alkyldimethylbenzylammoniumchloride, Dialkyldimethylammoniumchloride, Alkyltrimethylammoniumchloride und äthoxylierte quaternäre Ammoniumchloride.

Als Dispergiermittel (ohne Tensidwirkung) kommen im wesentlichen in Frage: Lignin, Natrium- und Ammoniumsalze von Ligninsulfonsäuren, Natriumsalze von Maleinsäureanhydrid-Diisobutylen-Copolymeren, Natrium- und Ammoniumsalze von sulfonierten Polykondensationsprodukten aus Naphthalin und Formaldehyd, und Sulfitablaugen.

Als Dispergiermittel, die sich insbesondere als Verdickungs bzw. Antiabsetzmittel eignen, können z.B. Methylcellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel, z.B. Epichlorhydrin, Phenylglycidäther und Soyaepoxide; Antioxidantien, z.B. Gallussäureester und Butylhydroxytoluol; UV-Absorber, z.B. substituierte Benzophenone, Diphenylacrylonitrilsäurester und Zimtsäureester; und Deaktivatoren, z.B. Salze der Aethylendiaminotetraessigsäure und Polyglykole.

Die erfindungsgemässen Unkrautbekämpfungsmittel können zusätzlich zu den erfindungsgemässen Wirkstoffen Synergisten und andere Wirkstoffe, z.B. Insektizide, Akarizide, Fungizide, Pflanzenwachstumsregulatoren und Düngemittel, enthalten. Solche Kombinationsmittel eignen sich zur Verstärkung der Aktivität bzw. zur Verbreiterung des Wirkungsspektrums.

Die erfindungsgemässen Unkrautbekämpfungsmittel enthalten im allgemeinen zwischen 0,001 und 95 Gewichtsprozent, vorzugsweise zwischen 0,5 und 75 Gewichtsprozent einer bzw. mehrerer erfindungsgemässer Verbindungen als Wirkstoff(e). Sie können z.B. in einer Form vorliegen, die sich für die Lagerung und den Transport eignet. In solchen Formulierungen, z.B. emulgierbaren Konzentraten, ist die Wirkstoffkonzentration normalerweise im höheren Bereich, vorzugsweise zwischen 1 und 50 Gewichtsprozent, insbesondere zwischen 10 und 30 Gewichtsprozent. Diese Formulierungen können dann, z.B. mit gleichen oder verschiedenen inerten Stoffen, bis zu Wirkstoffkonzentrationen verdünnt werden, die sich für den praktischen Gebrauch eignen, also vorzugsweise ca. 0,001 bis 10 Gewichtsprozent, insbesondere ca. 0,005 bis 5 Gewichtsprozent. Die Wirkstoffkonzentrationen können jedoch auch kleiner oder grösser sein.

Wie oben erwähnt, kann die Herstellung der erfindungsgemässen Unkrautbekämpfungsmittel in an sich bekannter Weise durchgeführt werden.

Zur Herstellung pulverförmiger Präparate kann der Wirkstoff, d.h. mindestens eine erfindungsgemässe Verbindung, mit festem Trägerstoff vermischt werden, z.B. durch Zusammenmahlen; oder man kann den festen Trägerstoff mit einer Lösung oder Suspension des Wirkstoffes imprägnieren und dann das Lösungsbzw. Dispersionsmittel durch Abdunsten, Erhitzen oder Absaugen unter vermindertem Druck entfernen. Durch Zusatz von Tensiden bzw. Dispergiermitteln kann man solche pulverförmige Mittel mit Wasser leicht benetzbar machen, so dass sie in wässrige Suspensionen, die sich z.B. als Spritzmittel eignen, übergeführt werden können.

Der Wirkstoff kann auch mit einem Tensid und einem festen Trägerstoff zur Bildung eines netzbaren Pulvers vermischt werden, welches in Wasser dispergierbar ist, oder er kann mit einem festen vorgranulierten Trägerstoff zur Bildung eines granulatförmigen Produktes vermischt werden.

Wenn gewünscht, kann der Wirkstoff in einem mit Wasser nicht mischbaren Lösungsmittel, wie beispielsweise einem hochsiedenden Kohlenwasserstoff, gelöst werden, das zweckmässigerweise gelösten Emulgator enthält, so dass die Lösung bei Zugabe zu Wasser selbstemulgierend wirkt. Andernfalls kann der Wirkstoff mit einem Emulgator vermischt und das Gemisch dann mit Wasser auf die gewünschte Konzentration verdünnt werden. Zudem kann der Wirkstoff in einem Lösungsmittel gelöst und danach mit einem Emulgator gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Die Verwendung der erfindungsgemässen Unkrautbekämpfungsmittel, die einen weiteren Gegenstand der vorliegenden Erfindung bildet, kann nach üblichen Applikationsmethoden, wie Spritzen, Sprühen, Stäuben, Giessen oder Streuen, erfolgen. Das erfindungsgemässe Verfahren zur Bekämpfung von Unkräutern ist dadurch gekennzeichnet, dass man das gegen Unkräuter zu schützende Gut und/oder die Unkräuter mit einer erfindungsgemässen Verbindung bzw. mit einem erfindungsgemässen Unkrautbekämpfungsmittel behandelt.

Die nachfolgenden Beispiele dienen zur näheren Erläuterung der Erfindung.

I. Herstellung der Wirkstoffe der Formel I:

Beispiel 1

2,5 g 2-Chlor-5-[3,6-dihydro-2,6-dioxo-3-methyl-4-trifluormethyl -1(2H)-pyrimidinyl]-4-fluorbenzoesäure werden in 20 ml Benzol und 2,9 ml Thionylchlorid zusammen mit 2 Tropfen Dimethylformamid 3 Stunden auf Rückflusstemperatur erhitzt. Anschliessend wird das Reaktionsgemisch zur Trockene eingedampft und in 20 ml Dioxan gelöst. Diese Lösung, die hauptsächlich aus dem Säurechlorid der obenerwähnten Benzoesäure und dem Lösungsmittel besteht, wird zu einer Lösung von 0,8 ml D,L-Milchsäure-methylester und 1,0 g Pyridin in 10 ml Dioxan getropft. Dann wird das Reaktionsgemisch 3 Stunden bei Raumtemperatur gerührt, mit 400 ml Wasser versetzt und zweimal mit je 400 ml Aethylacetat extrahiert. Man wäscht die vereinigten organischen Phasen zweimal mit je 200 ml Salzsäure und einmal mit 200 ml gesättigter Natriumchloridlösung, trocknet sie über wasserfreiem Natriumsulfat und dampft sie ein. Der Rückstand wird an Kieselgel mit Aethylacetat/n-Hexan (1:4) chromatographisch gereinigt. Auf diese erhält man den 2-{2-Chlor-5-[3,6-dihydro-2,6-dioxo-3-methyl -4-trifluormethyl-1(2H)-pyrimidinyl]-4-fluorbenzoyloxy} -propionsäure-methylester, der sich aus Aethylacetat/n-Hexan umkristallisieren lässt; Smp. 102-104 °C.

Beispiele 2-10

Analog dem in Beispiel 1 beschriebenen Verfahren erhält man aus 2-Chlor-5-[3,6-dihydro-2,6-dioxo-3-methyl-4-trifluormethyl -1(2H)-pyrimidinyl]-4-fluorbenzoesäure über deren Säurechlorid die in der nachstehenden Tabelle 1 aufgeführten Verbindungen der Formel I:

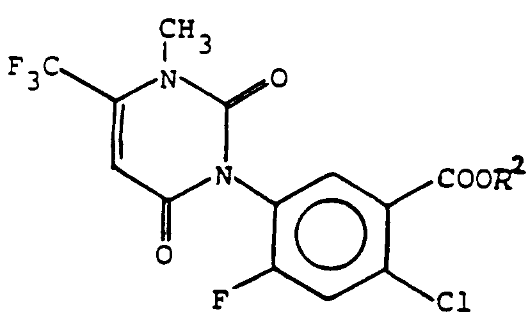

Tabelle 1

| Beispiel | $R^2$ | Physikalische Daten |
|---|---|---|
| 2 | Acetylmethyl | Oel;<br>$^1H$-NMR ($d_6$-DMSO, 400 MHz):<br>2,17 ppm (s,3H),<br>3,42 ppm (s,3H),<br>5,07 ppm (s,2H),<br>6,60 ppm (s,1H),<br>7,92 ppm (d,1H),<br>8,15 ppm (d,1H);<br>Massenspektrum (m/e):<br>422(18) $M^+$ |
| 3 | 1-Acetyläthyl | Oel;<br>$^1H$-NMR ($d_6$-DMSO, 400 MHz):<br>1,48 ppm (d,3H),<br>2,22 ppm (s,3H),<br>3,43 ppm (s,3H),<br>5,36 ppm (dq,1H),<br>6,61 ppm (d,1H),<br>7,92 ppm (d,1H),<br>8,14 ppm (d,1H) |

Tabelle 1 (Fortsetzung)

| | | |
|---|---|---|
| 4 | Trimethylsilylmethyl | Oel; $^1$H-NMR ($d_6$-DMSO, 400 MHz): 0,11 ppm (s,9H), 3,42 ppm (s,3H), 4,06 ppm (s,2H), 6,61 ppm (s,1H), 7,88 ppm (d,1H), 7,99 ppm (d,1H) |
| 5 | 2-(Trimethylsilyl)-äthyl | Oel;, $^1$H-NMR ($d_6$-DMSO, 400 MHz): 0,05-0,07 ppm (m,9H), 1,08-1,14 ppm (m,2H), 3,43 ppm (s,3H), 4,37-4,43 ppm (m,2H), 6,61 ppm (s,1H), 7,88 ppm (d,1H), 8,05 ppm (d,1H) |
| 6 | 2-(Methylsulfonyl)-äthyl | Smp. 140-142°C |
| 7 | 2-Phenoxyäthyl | Smp. 98-100°C |
| 8 | 2-Cyclohexenyl | Oel; $^1$H-NMR ($d_6$-DMSO, 400 MHz): 1,56-2,12 ppm (m,6H), 3,42 ppm (s,3H), 5,40-5,46 ppm (m,1H), 5,76-5,84 ppm (m,1H), 6,00-6,07 ppm (m,1H), 6,61 ppm (s,1H), 7,88 ppm (d,1H), 8,02 ppm (d,1H) |

Tabelle 1 (Fortsetzung)

| | | |
|---|---|---|
| 9 | Cyanomethyl | Smp. 113-115°C |
| 10 | [(1R,5S)-6,6-Dimethyl-2-norpinen-2-yl]-methyl | $^1$H-NMR ($d_6$-DMSO, 400 MHz): 0,80 ppm (s,3H), 1,13 ppm (d,1H), 1,26 ppm (s,3H), 2,06-2,12 ppm (m,1H), 2,16-2,25 ppm (m,2H), 2,26-2,36 ppm (m,1H), 2,37-2,44 ppm (m,1H), 3,53 ppm (s,breit,3H), 4,66-4,77 ppm (m,2H), 5,65-5,70 ppm (m,1H), 6,60 ppm (s,1H), 7,88 ppm (d,1H), 8,03 ppm (d,1H); $[\alpha]_D^{20}$ = -12,0° ($CHCl_3$, c = 0,4%) |

Beispiele 11-40

Analog dem in Beispiel 1 beschriebenen Verfahren erhält man ausgehend von 2-Chlor-5-[3,6-dihydro-3,4-dimethyl-2,6-dioxo-1(2H) -pyrimidinyl]-4-fluorbenzoesäure über deren Säurechlorid die in der nachstehenden Tabelle 2 aufgeführten Verbindungen der Formel I:

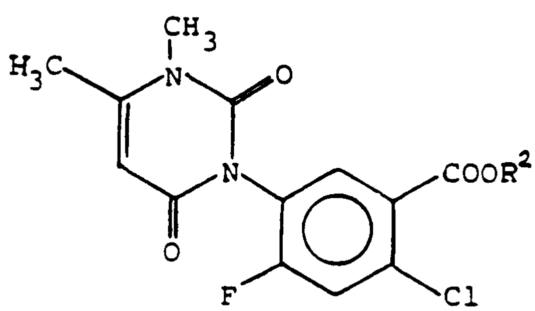

Tabelle 2

| Beispiel | $R^2$ | Physikalische Daten |
| --- | --- | --- |
| 11 | 2-Cyclohexenyl | Oel; $^1H$-NMR ($d_6$-DMSO, 400 MHz): 1,56-2,14 ppm (m,6H), 2,33 ppm (s,3H), 3,36 ppm (s,3H), 5,42 ppm (m,1H), 5,80 ppm (m,2H), 6,02 ppm (m,1H), 7,82 ppm (d,1H), 7,92 ppm (d,1H); Massenspektrum (m/e): 392(15) $M^+$ |

Tabelle 2 (Fortsetzung)

| | | |
|---|---|---|
| 12 | Propionylmethyl | Oel;<br>$^1H$-NMR ($d_6$-DMSO, 400 MHz):<br>0,98 ppm (t,3H),<br>2,33 ppm (s,3H),<br>2,51 ppm (d,2H),<br>3,35 ppm (s,3H),<br>5,06 ppm (s,2H),<br>5,80 ppm (s,1H),<br>7,87 ppm (d,1H),<br>8,03 ppm (d,1H);<br>Massenspektrum (m/e):<br>382(16) $M^+$ |
| 13 | Cyanomethyl | Oel;<br>$^1H$-NMR ($d_6$-DMSO, 400 MHz):<br>2,33 ppm (s,3H),<br>3,35 ppm (s,3H),<br>5,24 ppm (s,2H),<br>5,81 ppm (s,1H),<br>7,91 ppm (d,1H),<br>8,09 ppm (d,1H);<br>Massenspektrum (m/e):<br>351(21) $M^+$ |

Tabelle 2 (Fortsetzung)

| | | |
|---|---|---|
| 14 | Acetylmethyl | Oel; $^1$H-NMR ($d_6$-DMSO, 400 MHz): 2,17 ppm (s,3H), 2,33 ppm (s,3H), 3,35 ppm (s,3H), 5,05 ppm (s,2H), 5,81 ppm (s,1H), 7,87 ppm (d,1H), 8,03 ppm (d,1H); Massenspektrum (m/e): 368(36) $M^+$ |
| 15 | 2-Norbornanylmethyl (Gemisch aus endo-/exo-Form) | Oel; $^1$H-NMR ($d_6$-DMSO, 400 MHz): 0,70-1,90 ppm (m,8H), 2,06-2,28 ppm (m,3H), 2,33 ppm (s,3H), 3,35 ppm (s,3H), 4,00-4,37 ppm (m,2H), 5,80 ppm (s,1H), 7,83 ppm (d,1H), 7,92 ppm (dd,1H); Massenspektrum (m/e): 420(3,8) $M^+$ |

Tabelle 2 (Fortsetzung)

| | | |
|---|---|---|
| 16 | 1-(Methoxycarbonyl)-äthyl | Oel; $^{1}$H-NMR ($CDCl_3$, 60 MHz): 1,60 ppm (d,3H), 2,36 ppm (s,3H), 3,49 ppm (s,3H), 3,81 ppm (s,3H), 5,36 ppm (q,1H), 5,77 ppm (s,1H), 7,39 ppm (d,1H), 8,00 ppm (d,1H); Massenspektrum (m/e): 398(50) $M^+$ |
| 17 | 1-(Aethoxycarbonyl)-äthyl (R-Isomeres) | Oel; $^{1}$H-NMR ($CDCl_3$, 400 MHz): 1,27 ppm (t,3H), 1,59 ppm (d,3H), 2,32 ppm (s,3H), 3,44 ppm (s,3H), 4,22 ppm (q,2H), 5,29 ppm (q,1H), 5,73 ppm (s,1H), 7,35 ppm (d,1H), 7,95 ppm (dd,1H); Massenspektrum (m/e): 412(47) $M^+$; $[\alpha]_{Dnm}^{20°C}$=-19,36° ($CHCl_3$, c = 1,07%) |

Tabelle 2 (Fortsetzung)

| | | |
|---|---|---|
| 18 | 1-(Aethoxycarbonyl)-äthyl (S-Isomeres) | Oel; $^1$H-NMR ($CDCl_3$, 60 MHz): 1,29 ppm (t,3H), 1,64 ppm (d,3H), 2,36 ppm (s,3H), 3,50 ppm (s,3H), 4,29 ppm (q,2H), 5,48 ppm (q,1H), 5,81 ppm (s,1H), 7,44 ppm (d,1H), 8,04 ppm (d,1H); Massenspektrum (m/e): 412(39) $M^+$; $[\alpha]_{Dnm}^{20°C}$=+21,88° ($CHCl_3$, c = 0,99%) |
| 19 | n-Butoxycarbonylmethyl | Oel; $^1$H-NMR ($CDCl_3$, 60 MHz): 0,9-1,68 ppm (m,7H), 2,77 ppm (s,3H), 3,45 ppm (s,3H), 4,26 ppm (t,2H), 4,88 ppm (s,2H), 5,77 ppm (s,1H), 7,45 ppm (d,1H), 8,10 ppm (d,1H); Massenspektrum (m/e): 426(18) $M^+$ |

Tabelle 2 (Fortsetzung)

| | | |
|---|---|---|
| 20 | 1-[{2-[(α-Methyl-äthyliden)iminooxy]-äthyl}oxycarbonyl]-äthyl (R-Isomeres) | Oel; $^1H$-NMR ($CDCl_3$, 400 MHz): 1,59 ppm (dd,3H), 1,81 ppm (s,3H), 1,85 ppm (s,3H), 2,32 ppm (s,3H), 3,44 ppm (s,3H), 4,22 ppm (t,2H), 4,33-4,43 ppm (m,2H), 5,34 ppm (q,1H), 5,73 ppm (t,1H), 7,35 ppm (d,1H), 7,96 ppm (dd,1H); Massenspektrum (m/e): 483(10) $M^+$; $[\alpha]_{Dnm}^{20°C}$=-12,07° ($CHCl_3$, c = 1,07%) |
| 21 | 1-(Isobutyloxycarbonyl)-äthyl (R-Isomeres) | Oel; $^1H$-NMR ($CDCl_3$, 400 MHz): 0,92 ppm (d,6H), 1,60 ppm (d,3H), 1,95 ppm (q,1H), 2,32 ppm (s,3H), 3,43 ppm (s,3H), 3,90-3,99 ppm (m,2H), 5,32 ppm (q,1H), 5,73 ppm (s,1H), 7,36 ppm (d,1H), 7,96 ppm (q,1H); Massenspektrum (m/e): 440(12) $M^+$; $[\alpha]_{Dnm}^{20°C}$=-14,57° ($CHCl_3$, c = 1,07%) |

Tabelle 2 (Fortsetzung)

| | | |
|---|---|---|
| 22 | 1-(tert.Butoxycarbonyl)-äthyl | Oel;<br>$^1$H-NMR ($CDCl_3$, 400 MHz):<br>1,46 ppm (s,9H),<br>1,54 ppm (d,3H),<br>1,56 ppm (s,2H),<br>2,32 ppm (s,3H),<br>3,44 ppm (d,3H),<br>5,17 ppm (q,1H),<br>5,73 ppm (s,1H),<br>7,35 ppm (d,1H),<br>7,93 ppm (q,1H);<br>Massenspektrum (m/e):<br>440(5) $M^+$ |
| 23 | 1-(Propargyloxycarbonyl)-äthyl (R-Isomeres) | Oel;<br>$^1$H-NMR ($CDCl_3$, 400 MHz):<br>1,62 ppm (d,3H),<br>2,32 ppm (s,3H),<br>2,49 ppm (t,1H),<br>3,45 ppm (s,3H),<br>4,76 ppm (q,2H),<br>5,35 ppm (q,1H),<br>5,74 ppm (s,1H),<br>2,35 ppm (d,1H),<br>7,96 ppm (q,1H);<br>Massenspektrum (m/e):<br>422(22) $M^+$;<br>$[\alpha]_{Dnm}^{20°C}$=-16,05°<br>($CHCl_3$, c = 1,07%) |

Tabelle 2 (Fortsetzung)

| | | |
|---|---|---|
| 24 | 1-(Aethoxycarbonyl)-1-methyläthyl | Oel; $^1$H-NMR ($CDCl_3$, 400 MHz): 1,25 ppm (t,3H), 1,66 ppm (d,6H), 2,32 ppm (s,3H), 3,45 ppm (s,3H), 4,20 ppm (q,2H), 5,74 ppm (s,1H), 7,35 ppm (d,1H), 7,87 ppm (q,1H); Massenspektrum (m/e): 426(9) $M^+$ |
| 25 | Trimethylsilylmethyl | Harz; $^1$H-NMR ($CDCl_3$, 400 MHz): 0,13 ppm (s,9H), 2,33 ppm (s,3H), 3,45 ppm (s,3H), 4,01 ppm (s,2H), 5,74 ppm (s,1H), 7,33 ppm (d,1H), 7,82 ppm (d,1H) |
| 26 | 2-(Trimethylsilyl)-äthyl | Harz; $^1$H-NMR ($CDCl_3$, 400 MHz): 0,06 ppm (s,9H), 1,08-1,15 ppm (m,2H), 2,31 ppm (s,3H), 3,44 ppm (s,3H), 4,35-4,41 ppm (m,2H), 5,73 ppm (s,1H), 7,33 ppm (d,1H), 7,87 ppm (d,1H) |

Tabelle 2 (Fortsetzung)

| | | |
|---|---|---|
| 27 | Diäthylphosphonomethyl | Harz; $^1$H-NMR ($CDCl_3$, 400 MHz): 1,34 ppm (t,6H), 2,33 ppm (s,3H), 3,44 ppm (s,3H), 4,19 ppm (m,4H), 4,59 ppm (d,2H), 5,73 ppm (s,1H), 7,37 ppm (d,1H), 7,93 ppm (d,1H) |
| 28 | 2-(Diäthylphosphono)-äthyl | Harz; $^1$H-NMR ($CDCl_3$, 400 MHz): 1,32 ppm (t,6H), 2,23-2,33 ppm (m,2H), 2,33 ppm (s,3H), 3,44 ppm (s,3H), 4,06-4,18 ppm (m,4H), 4,50-4,58 ppm (m,2H), 5,73 ppm (s,1H), 7,36 ppm (d,1H), 7,91 ppm (d,1H) |
| 29 | 5-Norbornen-2-yl (Gemisch aus endo-/exo-Form) | Smp. 153-154°C |

Tabelle 2 (Fortsetzung)

| | | |
|---|---|---|
| 30 | 2-Cyanoäthyl | Oel; $^1H$-NMR ($d_6$-DMSO, 200 MHz): 2,36 ppm (s,3H), 3,05 ppm (t,2H), 3,36 ppm (s,3H), 4,49 ppm (t,2H), 5,83 ppm (s,1H), 7,88 ppm (d,1H), 7,98 ppm (d,1H); Massenspektrum (m/e): 365 (18), $M^+$ |
| 31 | 2-(Methylsulfonyl)-äthyl | Smp. 150-152°C |
| 32 | 2-Norbornanyl (exo-Form) | Oel; $^1H$-NMR ($CDCl_3$, 200 MHz): 1,04-1,27 ppm (m,3H), 1,36-1,66 ppm (m,5H), 1,73-1,88 ppm (m,1H), 2,32 ppm (s,3H), 2,40-2,48 ppm (m,1H), 3,45 ppm (s,3H), 4,78-4,87 ppm (m,1H), 5,74 ppm (s,1H), 7,33 ppm (d,1H), 7,80 ppm (d,1H); Massenspektrum (m/e): 406 (8,5), $M^+$ |

Tabelle 2 (Fortsetzung)

| | | |
|---|---|---|
| 33 | 2-Phenoxyäthyl | Oel; $^{1}$H-NMR ($CDCl_3$, 200 MHz): 2,31 ppm (s,3H), 3,43 ppm (s,3H), 4,24-4,32 ppm (m,2H), 4,60-4,68 ppm (m,2H), 5,73 ppm (s,1H), 6,88-7,00 ppm (m,3H), 7,23-7,40 ppm (m,3H), 7,87 ppm (d,1H); Massenspektrum (m/e): 432 (5,6), $M^+$ |
| 34 | 2-Allyloxyäthyl | Oel; $^{1}$H-NMR ($d_6$-DMSO, 200 MHz): 2,36 ppm (s,3H), 3,36 ppm (s,3H), 3,67-3,75 ppm (m,2H), 3,96-4,03 ppm (m,2H), 4,38-4,47 ppm (m,2H), 5,08-5,31 ppm (m,2H), 5,76-5,97 ppm (m,2H), 7,85 ppm (d,1H), 7,94 ppm (d,1H); Infrarot ($CHCl_3$): 1715 $cm^{-1}$ (νC=O), 1670 $cm^{-1}$ (νC=O) |

Tabelle 2 (Fortsetzung)

| | | |
|---|---|---|
| 35 | 2-(Methoxycarbonyl)-1-methyläthyl | Oel; $^1$H-NMR ($CDCl_3$), 200 MHz): 1,68 ppm (s,6H), 2,33 ppm (s,3H), 3,45 ppm (s,3H), 3,76 ppm (s,3H), 5,75 ppm (s,1H), 7,36 ppm (d,1H), 7,88 ppm (d,1H); Massenspektrum (m/e): 412 (10) $M^+$ |
| 36 | 2-(tert.Butoxycarbonyl)-1-methyläthyl | Oel; $^1$H-NMR ($CDCl_3$, 200 MHz): 1,45 ppm (s,9H), 1,64 ppm (s,6H), 2,33 ppm (s,3H), 3,45 ppm (s,3H), 5,54 ppm (s,1H), 7,65 ppm (d,1H), 7,80 ppm (d,1H); Massenspektrum (m/e): 454 (2), $M^+$ |

Tabelle 2 (Fortsetzung)

| | | |
|---|---|---|
| 37 | 1-Cyanoäthyl | Oel; $^1$H-NMR ($d_6$-DMSO, 200 MHz): 1,69 ppm (d,3H), 2,33 ppm (s,3H), 3,36 ppm (s,3H), 5,75 ppm (q,1H), 5,82 ppm (s,1H), 7,92 ppm (d,1H), 8,92 ppm (d,1H); Massenspektrum (m/e): 365 (28), $M^+$ |
| 38 | 1-(Aethoxycarbonyl)-1-methylpropyl | Oel; $^1$H-NMR ($CDCl_3$, 200 MHz): 0,98 ppm (t,3H), 1,26 ppm (t,3H), 1,68 ppm (d,3H), 1,64-2,36 ppm (m,2H), 2,33 ppm (d,3H), 3,46 ppm (s,3H), 4,08-4,26 ppm (m,2H), 5,75 ppm (d,1H), 7,35 ppm (d,1H), 7,84 ppm (d,1H); Massenspektrum (m/e): 440 (4), $M^+$ |

Tabelle 2 (Fortsetzung)

| | | |
|---|---|---|
| 39 | Methoxycarbonylmethyl | Oel; $^1$H-NMR ($CDCl_3$, 200 MHz): 2,33 ppm (s,3H), 3,45 ppm (s,3H), 3,79 ppm (s,3H), 4,84 ppm (d,2H), 5,74 ppm (s,1H), 7,38 ppm (d,1H), 8,00 ppm (d,1H); Massenspektrum (m/e): 384 (30), $M^+$ |
| 40 | 1-(Isopropoxycarbonyl)-äthyl (R)-Isomeres | Oel; $^1$H-NMR ($CDCl_3$, 400 MHz): 1,23-1,27 ppm (m,6H), 1,56-1,61 ppm (m,3H), 2,32 ppm (s,3H), 3,45 ppm (d,3H), 5,03-5,10 ppm (m,1H), 5,24 ppm (q,1H), 5,73 ppm (s,1H), 7,35 ppm (d,1H), 7,94 ppm (q,1H); $[\alpha]_D^{20}$ = -19,06° ($CHCl_3$, c = 0,61%) |

Beispiele 41-58

Analog dem in Beispiel 1 beschriebenen Verfahren erhält man aus 2-Chlor-5-[3,6-dihydro-2,6-dioxo-3-methyl-4-trifluormethyl-1(2H)-pyrimidinyl]-benzoesäure über deren Säurechlorid die in der nachstehenden Tabelle 3 aufgeführten Verbindungen der Formel I:

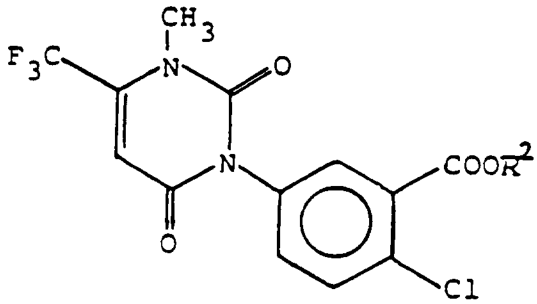

Tabelle 3

| Beispiel | $R^2$ | Physikalische Daten |
| --- | --- | --- |
| 41 | 2-Allyloxyäthyl | Oel; $^1H$-NMR ($d_6$-DMSO, 200 MHz): 3,41 ppm (s,3H), 3,64-3,76 ppm (m,2H), 3,96-4,03 ppm (m,2H), 4,40-4,48 ppm (m,2H), 5,09-5,32 ppm (m,2H), 5,77-5,97 ppm (m,1H), 6,56 ppm (s,1H), 7,54 ppm (dd,1H), 7,75 ppm (d,1H), 7,80 ppm (d,1H); Infrarot ($CHCl_3$): 1730 $cm^{-1}$ (νC=O), 1690 $cm^{-1}$ (νC=O) |

Tabelle 3 (Fortsetzung)

| | | |
|---|---|---|
| 42 | 2-Norbornanyl (exo-Form) | Smp. 126-127°C |
| 43 | 2-(Methoxycarbonyl)-1-methyläthyl | Smp. 156-157°C |
| 44 | 1-(Isopropoxycarbonyl)-äthyl (S-Isomeres) | $^1$H-NMR ($d_6$-DMSO, 200 MHz): 1,20 ppm (d,3H), 1,23 ppm (d,3H), 1,52 ppm (d,3H), 3,40 ppm (s,3H), 4,96 ppm (Septett,1H), 5,22 ppm (q,1H), 6,57 ppm (s,1H), 7,57 ppm (dd,1H), 7,78 ppm (d,1H), 7,86 ppm (d,1H); Massenspektrum (m/e): 462 (10), $M^+$; $[\alpha]_D^{20}$ = +14,4° ($CHCl_3$, c = 0,6%) |
| 45 | 1-(Aethoxycarbonyl)-1-methylpropyl (Racemat) | Smp. 135-137°C |
| 46 | 1-(tert.Butoxycarbonyl)-1-methyläthyl | Smp. 173-174°C |
| 47 | 1-(Allyloxycarbonyl)-1-methyläthyl | Smp. 109-111°C |
| 48 | 1-(Cyclopentyloxycarbonyl)-1-methyläthyl | Smp. 130-133°C |

Tabelle 3 (Fortsetzung)

| | | |
|---|---|---|
| 49 | 1-Carboxy-1-methyläthyl | Smp. 201-203°C |
| 50 | 2-Propargyloxyäthyl | Oel; $^1$H-NMR ($d_6$-DMSO, 200 MHz): 3,40 ppm (s,3H), 3,44 ppm (t,1H), 3,72-3,80 ppm (m,2H), 4,19 ppm (d,2H), 4,41-4,48 ppm (m,2H), 6,55 ppm (s,1H), 7,53 ppm (dd,1H), 7,75 ppm (d,1H), 7,78 ppm (d,1H); Massenspektrum (m/e): 430 (3), $M^+$ |
| 51 | 1-Methyl-2-phenoxy-äthyl (Racemat) | Oel; $^1$H-NMR ($d_6$-DMSO, 200 MHz): 1,42 ppm (d,3H), 3,40 ppm (s,3H), 4,10-4,26 ppm (m,2H), 5,36-5,50 ppm (m,1H), 6,56 ppm (s,1H), 6,86-7,01 ppm (m,3H), 7,19-7,32 ppm (m,2H), 7,51 ppm (dd,1H), 7,69 ppm (d,1H), 7,74 ppm (d,1H); Massenspektrum (m/e): 482 (5), $M^+$ |

Tabelle 3 (Fortsetzung)

| | | |
|---|---|---|
| 52 | 1-(Aethoxycarbonyl)-äthyl (S-Isomeres) | $^1$H-NMR ($d_6$-DMSO, 200 MHz): 1,21 ppm (t,3H), 1,53 ppm (d,3H), 3,40 ppm (s,3H), 4,17 ppm (q,2H), 5,28 ppm (q,1H), 6,35 ppm (s,1H), 7,56 ppm (q,1H), 7,77 ppm (d,1H), 7,88 ppm (d,1H); $[\alpha]_D^{20}$ = +12,4° ($CHCl_3$, c = 1,04%) |
| 53 | 1-(Aethoxycarbonyl)-1-methyläthyl | $^1$H-NMR ($d_6$-DMSO, 200 MHz): 1,19 ppm (t,3H), 1,62 ppm (s,6H), 3,40 ppm (s,1H), 4,16 ppm (q,2H), 6,55 ppm (s,1H), 7,54 ppm (d,1H), 7,76 ppm (d,1H), 7,80 ppm (d,1H); Massenspektrum (m/e):462 (4), $M^+$ |
| 54 | (2-Methylpropionyl)methyl | Smp. 127-129°C |

Tabelle 3 (Fortsetzung)

| | | |
|---|---|---|
| 55 | 1-Acetyläthyl | $^{1}$H-NMR ($CDCl_3$, 200 MHz): 1,53 ppm (d,3H), 2,25 ppm (s,3H), 3,56 ppm (q,3H), 5,33 ppm (q,1H), 6,38 ppm (s,1H), 7,33 ppm (q,1H), 7,62 ppm (d,1H), 7,84 ppm (d,1H); |
| 56 | 1-Acetyl-1-methyläthyl | Smp. 195-198°C |
| 57 | 2-Aethoxycarbonyl-1-methyläthyl | $^{1}$H-NMR ($CDCl_3$, 200 MHz): 1,23 ppm (t,3H), 1,42 ppm (d,3H), 2,60 ppm (q,1H), 2,80 ppm (q,1H), 3,55 ppm (q,3H), 4,14 ppm (q,2H), 5,55 ppm (m,1H), 6,38 ppm (s,1H), 7,29 ppm (q,1H), 7,59 ppm (d,1H), 7,71 ppm (d,1H); |
| 58 | 1-Cyano-1-methyläthyl | Smp. 182-184°C |

Beispiele 59-62

Analog dem in Beispiel 1 beschriebenen Verfahren erhält man aus der entsprechenden Verbindung der Formel IV über deren Säurechlorid die in der nachstehenden Tabelle 4 aufgeführten Verbindungen der Formel I:

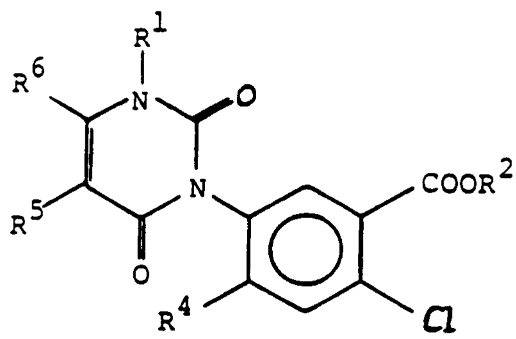

Tabelle 4

| Beispiel | $R^1$ | $R^2$ | $R^4$ | $R^5$ | $R^6$ | Physikalische Daten |
|---|---|---|---|---|---|---|
| 59 | $CH_3$ | 2-Allyl-oxyäthyl | F | H | $C_2F_5$ | $^1$H-NMR ($CDCl_3$, 200 MHz): 3,56-3,60 ppm (m,3H), 3,72-3,80 ppm (m,2H), 4,01-4,08 ppm (m,2H), 4,44-4,52 ppm (m,2H), 5,15-5,24 ppm (m,1H), 5,24-5,36 ppm (m,1H), 5,80-6,01 ppm (m,1H), 6,36 ppm (s,1H), 7,40 ppm (d,1H), 7,93 ppm (d,1H); Infrarot ($CHCl_3$): 1735 $cm^{-1}$ (νC=O), 1680 $cm^{-1}$ (νC=O) |

Tabelle 4 (Fortsetzung)

| | | | | | | |
|---|---|---|---|---|---|---|
| 60 | $CHF_2$ | 2-Allyl-<br>oxyäthyl | F | H | $CH_3$ | $^1$H-NMR (d$_6$-DMSO,<br>200 MHz):<br>2,43 ppm (s,3H),<br>3,66-3,74 ppm (m,2H),<br>3,97-4,03 ppm (m,2H),<br>4,40-4,47 ppm (m,2H),<br>5,09-5,18 ppm (m,1H),<br>5,18-5,31 ppm (m,1H),<br>5,78-5,97 ppm (m,1H),<br>6,08 ppm (s,1H),<br>7,90 ppm (d,1H),<br>7,90 ppm (t,1H),<br>8,11 ppm (d,1H);<br>Infrarot ($CHCl_3$):<br>1735 cm$^{-1}$ (νC=0),<br>1695 cm$^{-1}$ (νC=0) |
| 61 | $CHF_2$ | 1-(Aeth-<br>oxycarbonyl)-<br>1-methyläthyl | H | H | $CH_3$ | Smp. 91-93°C |
| 62 | $CH_3$ | 2-Allyl-<br>oxyäthyl | F | F | $CF_3$ | $^1$H-NMR (d$_6$-DMSO,<br>200 MHz):<br>3,40-3,44 ppm (m,3H),<br>3,67-3,74 ppm (m,2H),<br>3,96-4,03 ppm (m,2H),<br>4,41-4,48 ppm (m,2H),<br>5,10-5,18 ppm (m,1H),<br>5,18-5,32 ppm (m,1H),<br>5,78-7,98 ppm (m,1H),<br>7,95 ppm (d,1H),<br>8,06 ppm (d,1H);<br>Infrarot ($CHCl_3$):<br>1760 cm$^{-1}$ (νC=0),<br>1690 cm$^{-1}$ (νC=0) |

Beispiel 63

Analog dem in Beispiel 1 beschriebenen Verfahren erhält man aus 2-Chlor-4-fluor-5-[2-methoxy-6-oxo-4-trifluormethyl-1(6H)-pyrimidinyl]-benzoesäure über deren Säurechlorid und 2-Allyloxyäthanol den 2-Chlor-4-fluor-5-[2-methoxy-6-oxo-4-trifluormethyl-1(6H)-pyrimidinyl]-benzoesäure-(2-allyloxyäthyl)ester, Smp. 74-77°C.

Beispiel 64

Ein Gemisch aus 1,5 g 2-Chlor-5-[3,6-dihydro-3,4-dimethyl-2,6-dioxo-1(2H)-pyrimidinyl]-4-fluorbenzoeäure-(1-methoxycarbonyl-1-methyläthyl)ester und 0,4 g Kupfer(I)cyanid wird in 20 ml 1,3-Dimethyl-3,4,5,6-tetrahydro-2(1H)-pyrimidinon unter Stickstoff 6 Stunden auf 220°C erhitzt. Nach Abkühlen auf Raumtemperatur wird das Gemisch filtriert und das Filtrat in 200 ml Aethylacetat gelöst. Man wäscht die Lösung viermal mit je 50 ml Wasser, trocknet die vereinigten organischen Phasen und engt sie unter vermindertem Druck ein. Auf diese Weise erhält man den 2-Cyano-5-[3,6-dihydro-3,4-dimethyl-2,6-dioxo-1(2H)-pyrimidinyl]-4-fluorbenzoesäure-(1-methoxycarbonyl-1-methyläthyl)ester, Massenspektrum (m/e): 403 (10) $M^+$.

Beispiel 65

Analog dem in Beispiel 64 beschriebenen Verfahren erhält man aus 2-Chlor-5-[3,6-dihydro-3,4-dimethyl-2,6-dioxo-1(2H)-pyrimidinyl]-4-fluorbenzoesäure-(1-cyanoäthyl)ester und Kupfer(I)cyanid den 2-Cyano-5-[3,6-dihydro-3,4-dimethyl-2,6-dioxo-1(2H)-pyrimidinyl]-4-fluorbenzoesäure-(1-cyanoäthyl)ester, Massenspektrum (m/e): 356 (17) $M^+$.

Beispiel 66

4,9 g Natriumhydrid (50% Dispersion in Weissöl) werden unter Rühren zu 120 ml Dimethylformamid gegeben, und das Gemisch wird auf -5°C abgekühlt. Bei dieser Temperatur wird eine Lösung von 22,4 g 3-Amino-4,4,4-trifluorcrotonsäure-äthylester in 180 ml Diäthyläther während 30 Minuten zugetropft. Das Reaktionsgemisch wird eine Stunde bei Raumtemperatur nachgerührt und danach auf -65°C abgekühlt. Nun wird eine Lösung von 28 g 2-(2-Chlor-5-isocyanato-benzoyloxy) -2-methylpropionsäure-äthylester in 100 ml Dimethylformamid während 30 Minuten zugetropft. Das Reaktionsgemisch wird 2 Stunden bei -65°C nachgerührt; danach wird es auf Raumtemperatur erwärmen gelassen. Das Reaktionsgemisch wird in 300 ml 1N Salzsäure bei 0°C eingetragen. Es wird zweimal mit je 300 ml Essigester extrahiert. Die organischen Phasen werden einmal mit 200 ml Wasser und einmal mit 200 ml gesättigter Natriumchloridlösung nachgewaschen, über wasserfreiem Natriumsulfat getrocknet und unter vermindertem Druck eingeengt. Der Rückstand wird aus Essigester/n-Hexan umkristallisiert. Man erhält den 2-{2-Chlor-5-[3,6-dihydro-2,6-dioxo -4-trifluormethyl-1(2H)-pyrimidinyl]-benzoyloxy} -2-methyl-propionsäure-äthylester, Smp. 176-177°C.

Beispiel 67

Analog dem in Beispiel 66 beschriebenen Verfahren erhält man aus 3-Amino-4,4,5,5,5,-pentafluor-pent -2-ensäure-äthylester und 2-(2-Chlor-5-isocyanato-benzyloxy) -2-methyl-propionsäure-äthylester den 2-{2-Chlor-5-[3,6-dihydro 2,6-dioxo-4-pentafluoräthyl-1(2H)-pyrimidinyl]-benzyloxy} -2-methyl-propionsäure-äthylester, Smp. 141-143°C.

Beispiel 68

2,5 g 2-{2-Chlor-5-[3,6-dihydro-2,6-dioxo -4-pentafluoräthyl-1(2H)-pyrimidinyl]-benzoyloxy} -2-methyl-propionsäure-äthylester und 0,7 g Kaliumcarbonat werden in 25 ml Wasser und 15 ml Dioxan gelöst. Es wird 0,65 g Dimethylsulfat hinzugefügt, und das Reaktionsgemisch wird 38 Stunden bei Raumtemperatur gerührt. Danach werden 100 ml Wasser hinzugefügt; und es wird zweimal mit je 200 ml Essigester extrahiert. Die organischen Phasen werden einmal mit 150 ml gesättigter Kochsalzlösung nachgewaschen, über wasserfreiem Natriumsulfat getrocknet und unter vermindertem Druck eingeengt. Der Rückstand wird an Kieselgel mit Essigester/n-Hexan (1:4) chromatographisch gereinigt. Man erhält so den 2-{2-Chlor-5-[3,6-dihydro-2,6-dioxo -3-methyl-4-pentafluoräthyl-1(2H)-pyrimidinyl]-benzoyloxy} -2-methyl-propionsäure-äthylester, der aus Diäthyläther/n-Hexan umkristallisiert wird; Smp. 114-115°C.

Beispiel 69

Analog dem in Beispiel 68 beschriebenen Verfahren erhält man aus 2-{2-Chlor-5-[3,6-dihydro-2,6-dioxo -4-trifluormethyl-1(2H)-pyrimidinyl]-benzyloxy} -2-methyl-propionsäure-äthylester und Diäthylsulfat den 2-{2-Chlor-5-[3,6-dihydro-2,6-dioxo -3-äthyl-4-trifluormethyl-1(2H)-pyrimidinyl]-benzoyloxy} -2-methyl-propionsäure-äthylester als Oel;
$^1$H-NMR ($d_6$-DMSO, 200 MHz): 1,20 ppm (t, 3H), 1,24 ppm (t, 3H), 1,62 ppm (s, 6H), 3,87 ppm (q, 2H), 4,16 ppm (q, 2H), 6,55 ppm (s, 1H), 7,57 ppm (dd, 1H), 7,75 ppm (d, 1H), 7,82 ppm (d, 1H).

Beispiel 70

0,35 g Natriumhydrid (50% Dispersion in Weissöl) wird zu 40 ml Dimethylformamid unter Rühren gegeben. Es werden 2,9 g 2-{2-Chlor-5-[3,6-dihydro-2,6-dioxo -4-trifluormthyl-1(2H)-pyrimidinyl]-benzoyloxy} -2-methyl-propionsäure-äthylester portionenweise als Festkörper zugegeben. Nach beendeter Zugabe wird noch eine Stunde bei Raumtemperatur nachgerührt. Nun werden 1,2 g Allylbromid in einer Portion zugegeben. Das Reaktionsgemisch wird 18 Stunden bei Raumtemperatur gerührt und danach in 100 ml Wasser bei 0 °C eingetragen. Es wird zweimal mit je 150 ml Essigester extrahiert. Die organischen Phasen werden einmal mit 100 ml gesättigter Natriumchloridlösung gewaschen, über wasserfreiem Natriumsulfat getrocknet und unter vermindertem Druck eingeengt. Der Rückstand wird über Kieselgel mit Essigester/n-Hexan (1:4) chromatographisch gereinigt. Man erhält den 2-{2-Chlor-5-[3,6-dihydro-2,6-dioxo -3-(2-propenyl)-4-trifluormethyl-1(2H)-pyrimidinyl]-benzoyloxy} -2-methyl-propionsäure-äthylester als Oel;
$^1$H-NMR ($d_6$-DMSO, 200 MHz): 1,19 ppm (t, 3H), 1,62 ppm (s, 6H), 4,15 ppm (q, 2H), 4,42-4,53 ppm (m, 2H), 5,14-5,34 ppm (m, 2H), 5,78-6,00 ppm (m, 1H), 6,60 ppm (s, 1H), 7,58 ppm (dd, 1H), 7,75 ppm (d, 1H), 7,83 ppm (d, 1H).

Beispiel 71

Analog dem in Beispiel 70 beschriebenen Verfahren erhält man aus 2-{2-Chlor-5-[3,6-dihydro-2,6-dioxo -4-trifluormethyl-1(2H)-pyrimidinyl]-benzyloxy} -2-methyl-propionsäure-äthylester und Propargylbromid den 2-{2-Chlor-5-[3,6-dihydro-2,6-dioxo -3-(3-propinyl)-4-trifluormethyl-1(2H)-pyrimidinyl] -benzoyloxy} -2-methyl-propionsäure-äthylester als Oel,
$^1$H-NMR ($d_6$-DMSO, 200 MHz): 1,19 ppm (t, 3H), 1,62 ppm (s, 6H), 3,43 ppm (t, 1H), 4,16 ppm (q, 2H), 4,63 ppm (d, 2H), 6,64 ppm (s, 1H), 7,60 ppm (dd, 1H), 7,76 ppm (d, 1H), 7,85 ppm (d, 1H).

Beispiel 72

Ein Gemisch von 9 g 2-[2-Chlor-4-fluor-5-(3-oxo-pentanoyl)-aminobenzoyloxy] -2-methyl-propionsäure-äthylester, 2,7 g Urethan und 0,9 g p-Toluolsulfonsäure wird in 150 ml Benzol erhitzt. Während der fünfstündigen Reaktionszeit wird das in der Reaktion entwickelte Wasser abgeschieden. Das Lösungsmittel wird dann unter vermindertem Druck abgedampft. Man erhält so den rohen 2-{2-Chlor-4-fluor-5-[3-(äthoxycarbonyl)-amino-pent -2-enoyl]-amino-benzoyloxy} -2-methyl-propionsäure-äthylester.

0,6 g Natrium wird in 50 ml α-Hydroxyisobuttersäure-äthylester vollständig aufgelöst. Diese Lösung wird mit dem rohen 2-{2-Chlor-4-fluor -5-[3-(äthoxycarbonyl)-amino-pent -3-enoyl]-amino-benzoyloxy}-2-methyl-propionsäure-äthylester vereinigt und 4 Stunden bei 120 °C gerührt. Nach Erkalten wird dieses Reaktionsgemisch in 100 ml 1N Salzsäure bei 0 °C eingetragen. Es wird zweimal mit je 150 ml Essigester extrahiert, zweimal mit je 200 ml Wasser nachgewaschen, über wasserfreiem Magnesiumsulfat getrocknet und unter vermindertem Druck eingeengt. Der Rückstand wird an Kieselgel mit Essigester/n-Hexan (3:2) chromatographisch gereinigt. Man erhält den 2-{2-Chlor-4-fluor-5-[3,6-dihydro-2,6-dioxo -4-äthyl-1(2H)-pyrimidinyl]-benzoyloxy} -2-methyl-propionsäure-äthylester, der aus Diäthyläther/n-Hexan umkristallisiert wird; Smp. 166-167 °C.

Beispiel 73

Ein Gemisch von 1,2 g 2-{2-Chlor-4-fluor-5-[3,6-dihydro-2,6-dioxo -4-äthyl-1(2H)-pyrimidinyl]-benzoyloxy} -2-methyl-propionsäure-äthylester, 0,4 g Dimethylsulfat und 0,7 g Kaliumhydrogencarbonat wird 3 Stunden in 30 ml Aceton auf Rückflusstemperatur erhitzt. Das Reaktionsgemisch wird mit 200 ml Wasser versetzt und zweimal mit je 150 ml Essigester extrahiert. Die organischen Phasen werden einmal mit 100 ml gesättigter Natriumchloridlösung gewaschen, über wasserfreiem Natriumsulfat getrocknet und unter

vermindertem Druck eingeengt. Der Rückstand wird über Kieselgel mit Essigester/n-Hexan (3:2) chromatographisch gereinigt. Man erhält so den 2-{2-Chlor-4-fluor-5-[3,6-dihydro-2,6-dioxo -3-methyl-4-äthyl-1(2H)-pyrimidinyl]-benzoyloxy} -2-methyl-propionsäure-äthylester als Oel, $^1$H-NMR ($d_6$-DMSO): 1,19 ppm (t, 6H), 1,61 ppm (s, 6H), 2,66 ppm (q, 2H), 3,37 ppm (s, 3H), 4,15 ppm (q, 2H), 5,72 ppm (s, 1H), 7,86 ppm (d, 1H), 7,99 ppm (d, 1H).

II. Herstellung der Ausgangsmaterialien der Formeln XII, XV und XVIII

Beispiel 74

10 g 2-Chlor-4-fluor-5-nitro-benzoesäure, 5 ml Thionylchlorid und 2 Tropfen N,N-Dimethylformamid werden 4 Stunden auf Rückflusstemperatur erhitzt. Anschliessend wird das Reaktionsgemisch zur Trockene eingedampft und in 20 ml Dioxan gelöst. Diese Lösung, die hauptsächlich aus dem Säurechlorid der oben erwähnten Benzoesäure und dem Lösungsmittel besteht, wird zu einer Lösung von 9,1 g α-Hydroxy-isobuttersäure-äthylester, 7 ml Triäthylamin und 100 mg 4-(Dimethylamino)-pyridin in 35 ml Dioxan getropft. Dann wird das Reaktionsgemisch 24 Stunden auf Rückflusstemperatur erhitzt. Sodann wird das Reaktionsgemisch erkalten gelassen und mit 200 ml 2N Salzsäure versetzt. Es wird nun dreimal mit je 250 ml Essigester extrahiert. Man wäscht die organischen Phasen einmal mit 200 ml 2N Salzsäure, einmal mit 200 ml Wasser, einmal mit 200 ml 2N Kaliumhydrogencarbonatlösung und zweimal mit je 200 ml gesättigter Kochsalzlösung, trocknet sie über wasserfreiem Magnesiumsulfat und dampft sie unter vermindertem Druck ein. Der Rückstand wird über Kieselgel mit Essigester/n-Hexan (1:4) chromatographisch gereinigt. Man erhält auf diese Weise den 2-(2-Chlor-4-fluor-5-nitro-benzoyloxy) -2-methyl-propionsäure-äthylester, der aus Diisopropyläther/n-Hexan umkristallsiert wird; Smp. 55-57 °C.

Ein Gemisch von 10 g fein gepulvertes Eisen, 50 ml Aethanol, 11 ml Wasser und 1 ml konzentrierter Salzsäure wird unter Rühren auf 65 °C erwärmt. Zu diesem Gemisch wird eine Lösung von 15 g 2-(2-Chlor-4-fluor-5-nitro-benzoyloxy) -2-methyl-propionsäure-äthylester in 15 ml Aethanol während 2 Stunden so zugetropft, dass die Temperatur nicht über 80 °C steigt. Nach beendeter Zugabe wird das Reaktionsgemisch weitere 16 Stunden bei 70 °C gerührt. Nach Erkalten wird das Reaktionsgemisch über Celite® abgenutscht. Das Celite® wird mit Wasser und mit Essigester gut nachgewaschen. Das Filtrat wird mit 2N Kaliumhydrogencarbonatlösung auf pH 8 eingestellt. Es wird nochmals über Celite® filtriert und erneut mit Wasser und Essigester nachgewaschen. Das Filtrat wird nun dreimal mit je 400 ml Essigester extrahiert. Die organischen Phasen werden zweimal mit je 300 ml gesättigter Natriumchloridlösung nachgewaschen, über wasserfreiem Magnesiumsulfat getrocknet und unter vermindertem Druck eingedampft. Der Rückstand wird am Hochvakuum fraktioniert und destilliert. Man erhält auf diese Weise den 2-(5-Amino-2-chlor-4-fluor-benzoyloxy) -2-methyl-propionsäure-äthylester, Sdp. 146-151 °C/0,07 Torr.

Beispiel 75

Analog dem in Beispiel 74 beschriebenen Verfahren erhält man aus 2-Chlor-5-nitro-benzoesäure über deren Säurechlorid und α-Hydroxy-isobuttersäure-äthylester den 2-(2-Chlor-5-nitro-benzoyloxy) -2-methyl-propionsäure-äthylester, Smp. 64-66 °C, den man anschliessend zum 2-(5-Amino-2-chlor-benzoyloxy) -2-methyl-propionsäure-äthylester reduziert. Sdp. 200-230 °C/0,1 Torr;
$^1$H-NMR ($CDCl_3$, 200 MHz): 1,28 ppm (t, 3H), 1,69 ppm (s, 6H), 3,79 ppm (s, 2H), 4,23 ppm (q, 2H), 6,72 ppm (dd, 1H), 7.10 ppm (d, 1H), 7,19 ppm (d, 1H).

Beispiel 76

Eine Lösung von 40 g Diphosgen in 150 ml Essigester wird unter Rühren auf 65 °C erwärmt. Zu dieser Lösung wird eine Lösung von 40 g 2-(5-Amino-2-chlor-benzoyloxy) -2-methyl-propionsäure-äthylester in 70 ml Essigester zugetropft. Nach beendeter Zugabe wird das Reaktionsgemisch 3 Stunden auf Rückflusstemperatur erhitzt. Danach wird das Lösungsmittel abdestilliert. Der Rückstand wird im Kugelrohr bei 200-230 °C und 0,1 Torr destilliert. Man erhält auf diese Weise den 2-(2-Chlor-5-isocyanato-benzoyloxy) -2-methyl-propionsäure-äthylester,
$^1$H-NMR ($CDCl_3$, 200 MHz): 1,29 ppm (t, 3H), 1,71 ppm (s, 6H), 4,25 ppm (q, 2H), 7,14 ppm (dd, 1H), 7,40 ppm (d, 1H), 7,52 ppm (d, 1H).

Beispiel 77

5,7 g 2,2-Dimethyl-1,3-dioxan-4,6-dion ("Meldrum's Säure") und 6,3 ml Pyridin werden in 25 ml Methylenchlorid vorgelegt, und das Gemisch wird unter Rühren auf 0 °C abgekühlt. Bei dieser Temperatur werden 3,8 ml Propionsäurechlorid während 30 Minuten zugetropft. Das Reaktionsgemisch wird sodann eine Stunde bei Raumtemperatur nachgerührt und danach in 100 ml 1N Salzsäure bei 0 °C eingetragen. Es wird rasch zweimal mit je 250 ml Diäthyläther extrahiert. Die organischen Phasen werden zweimal mit je 150 ml gesättigter Kochsalzlösung gewaschen, über wasserfreiem Magnesiumsulfat getrocknet und eingeengt. Der Rückstand wird in 50 ml Toluol aufgenommen. Es werden 12 g 2-(5-Amino-2-chlor-4-fluor-benzoyloxy) -2-methyl-propionsäure-äthylester hinzugefügt und das Ganze 4 Stunden auf Rückflusstemperatur erhitzt. Das Reaktionsgemisch wird 16 Stunden gerührt, wobei es sich auf Raumtemperatur abkühlte. Das Reaktionsgemisch wird in 150 ml 1N Salzsäure eingetragen. Es wird dreimal mit je 250 ml Diäthyläther extrahiert. Die organischen Phasen werden zweimal mit je 150 ml gesättigter Kochsalzlösung nachgewaschen, über wasserfreiem Magnesiumsulfat getrocknet und unter vermindertem Druck eingeengt. Der Rückstand wird an Kieselgel mit Essigester/n-Hexan (1:1) chromatographisch gereinigt. Auf diese Weise erhält man den 2-[2-Chlor-4-fluor-5-(3-oxo-pentanoyl)-amino-benzoyloxy] -2-methyl-propionsäure-äthylester als Oel,
$^{1}$H-NMR ($CDCl_3$, 200 MHz): 1,14 ppm (t, 3H), 1,27 ppm (t, 3H), 1,70 ppm (s, 6H), 2,63 ppm (q, 2H), 3,63 ppm (s, 2H), 4,23 ppm (q, 2H), 7,24 ppm (d, 1H), 8,82 ppm (d, 1H), 9,70 ppm (s, 1H).

III. Formulierungsbeispiele:

Beispiel 78

Ein emulgierbares Konzentrat enthält folgende Bestandteile:

| | |
|---|---|
| Erfindungsgemässe Verbindung (Wirkstoff) | 50 g/l |
| N-Methylpyrrolidon (Lösungsvermittler) | 200 g/l |
| Nonylphenol-(10)äthoxylat (nichtionogener Emulgator) | 50 g/l |
| Calcium-dodecylbenzolsulfonat (anionischer Emulgator) | 25 g/l |
| Gemisch von Alkylbenzolen (Lösungsmittel) | ad 1000 ml |

Der Wirkstoff und die Emulgatoren werden unter Rühren im Lösungsvermittler gelöst, und die Lösung wird mit dem Lösungsmittel auf 1 Liter ergänzt.

Das resultierende emulgierbare Konzentrat lässt sich in Wasser emulgieren und ergibt so eine gebrauchsfertige Spritzbrühe mit der gewünschten Konzentration.

Beispiel 79

Zur Herstellung eines 25% Spritzpulvers werden die nachstehend aufgeführten Bestandteile miteinander vermischt:

| | |
|---|---|
| Erfindungsgemässe Verbindung (Wirkstoff) | 25 g |
| Kieselsäure, hydratisiert (Trägerstoff, Mahlhilfsmittel) | 5 g |
| Natrium-laurylsulfat (Netzmittel) | 1 g |
| Natrium-lignosulfonat (Dispergator) | 2 g |
| Kaolin (Trägerstoff) | 67 g |
| | 100 g |

Anschliessend wird das Gemisch unter Verwendung einer Stiftmühle oder vergleichbaren Mahleinrichtung fein gemahlen.

Das resultierende Spritzpulver ergibt beim Einrühren in Wasser eine feine Suspension, die sich als gebrauchsfertige Spritzbrühe eignet.

**Patentansprüche**

**1.** Verbindungen der allgemeinen Formel

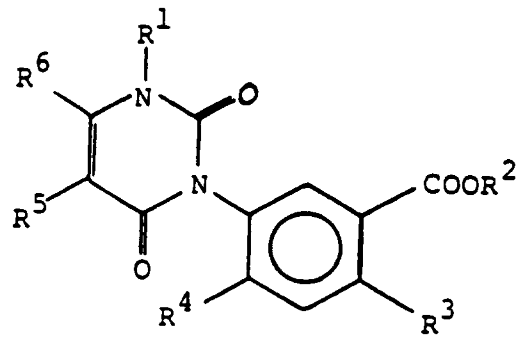

I

worin

| | |
|---|---|
| $R^1$ | Wasserstoff, $C_{1-4}$-Alkyl, $C_{3\ \text{oder}\ 4}$-Alkenyl, $C_{3\ \text{oder}\ 4}$-Alkinyl oder $C_{1-4}$-Halogenalkyl, |
| $R^2$ | $C_{1-6}$-Alkylsulfonyl-$C_{1-4}$-alkyl, Di($C_{1-6}$-alkyl)phosphono-$C_{1-4}$-alkyl, Tri($C_{1-6}$-alkyl)-silyl-$C_{1-4}$-alkyl, $C_{2-7}$-Alkanoyl-$C_{1-4}$-alkyl, Carboxy-$C_{1-4}$-alkyl, ($C_{1-6}$-Alkoxy)-carbonyl-$C_{1-4}$-alkyl, {[$C_{3-9}$-($\alpha$-Alkylalkyliden)]iminooxy -($C_{1-6}$-alkoxy)carbonyl}-$C_{1-4}$-alkyl, ($C_{3-6}$-Alkenyloxy)carbonyl-$C_{1-4}$-alkyl, ($C_{3-6}$-Alkinyloxy)carbonyl-$C_{1-4}$-alkyl, ($C_{3-8}$-Cycloalkyloxy)carbonyl-$C_{1-4}$-alkyl, Phenoxy-$C_{1-4}$-alkyl, $C_{3-6}$-Alkenyloxy-$C_{1-4}$-alkyl, $C_{3-8}$-Cycloalkenyloxy-$C_{1-}$ $_{-4}$-alkyl, $C_{5-8}$-Cycloalkenyl, $C_{6-8}$-Bicycloalkyl, $C_{6-8}$-Bicycloalkenyl, $C_{2-7}$-Cyanoalkyl oder $C_{3-6}$-Alkinyloxy-$C_{1-4}$-alkyl, |
| $R^3$ | Halogen oder Cyano, |
| $R^4$ | Wasserstoff oder Halogen, |
| $R^5$ | Wasserstoff, Fluor oder $C_{1-4}$-Alkyl und |
| $R^6$ | $C_{1-4}$-Alkyl oder $C_{1-4}$-Halogenalkyl bedeuten, oder |
| $R^5$ und $R^6$ | zusammen Tri- oder Tetramethylen bilden, |

und die entsprechenden Enoläther derjenigen Verbindungen der Formel I, in denen $R^1$ von Wasserstoff oder $C_{1-4}$-Halogenalkyl verschieden ist, sowie Salze derjenigen Verbindungen der Formel I, in denen $R^1$ Wasserstoff bedeutet.

**2.** Verbindungen nach Anspruch 1, worin $R^2$ verschieden von Carboxy-$C_{1-4}$-alkyl ist, $R^5$ Wasserstoff, Fluor oder $C_{1-4}$-Alkyl bedeutet und $R^6$ $C_{1-4}$-Alkyl oder $C_{1-4}$-Halogenalkyl bedeutet, sowie deren Enoläther und Salze.

**3.** Verbindungen nach Anspruch 1 oder 2, worin $R^1$ Methyl, $R^3$ Chlor oder Brom, $R^4$ Wasserstoff oder Fluor, $R^5$ Wasserstoff, Fluor oder Methyl und $R^6$ Methyl, Trifluormethyl oder Pentafluormethyl bedeuten.

**4.** Verbindungen nach Anspruch 1, worin $R^3$ Cyano bedeutet.

**5.** Verbindungen nach einem der Ansprüche 1 bis 4, worin $R^2$ $C_{2-7}$-Alkanoyl-$C_{1-4}$-alkyl, ($C_{1-6}$-Alkoxy)-carbonyl-$C_{1-4}$-alkyl, {[$C_{3-9}$-($\alpha$-Alkylalkyliden)]iminooxy-($C_{1-6}$-alkoxy)carbonyl}-$C_{1-4}$-alkyl, ($C_{3-6}$-Alkenyloxy)carbonyl-$C_{1-4}$-alkyl, ($C_{3-6}$-Alkinyloxy)carbonyl-$C_{1-4}$-alkyl oder ($C_{3-8}$-Cycloalkyloxy)carbonyl-$C_{1-4}$ -alkyl bedeutet.

**6.** Eine Verbindung nach Anspruch 2, 2-{2-Chlor-5-[3,6-dihydro-3,4-dimethyl-2,6-dioxo-1(2H)-pyrimidinyl]-4-fluorbenzoyloxy}-2-methyl-propionsäure-äthylester.

**7.** Eine Verbindung nach Anspruch 1, ausgewählt aus:

2-{2-Chlor-5-[3,6-dihydro-3,4-dimethyl-2,6-dioxo-1(2H)-pyrimidinyl]-4-fluorbenzoyloxy}-2-methyl-propionsäure-methylester,

2-{2-Chlor-5-[3,6-dihydro-3,4-dimethyl-2,6-dioxo-1(2H)-pyrimidinyl]-4-fluorbenzoyloxy}-2-methyl-propionsäure-(tert.butyl)ester,

2-Aethyl-2-{2-chlor-5-[3,6-dihydro-3,4-dimethyl-2,6-dioxo-1(2H)-pyrimidinyl]-4-fluorbenzoyloxy}-propionsäure-äthylester,

2-{2-Chlor-5-[3,6-dihydro-2,6-dioxo-3-methyl-4-trifluormethyl-1(2H)-pyrimidinyl]-benzoyloxy}-2-methyl-propionsäure-methylester,

2-{2-Chlor-5-[3,6-dihydro-2,6-dioxo-3-methyl-4-trifluormethyl-1(2H)-pyrimidinyl]-benzoyloxy}-propionsäure-isopropylester,

2-Aethyl-2-{2-chlor-5-[3,6-dihydro-2,6-dioxo-3-methyl-4-trifluormethyl-1(2H)-pyrimidinyl]-benzoyloxy}-propionsäure-äthylester,

2-{2-Chlor-5-[3,6-dihydro-2,6-dioxo-3-methyl-4-trifluormethyl-1(2H)-pyrimidinyl]-benzoyloxy]-2-methyl-propionsäure-(tert.butyl)ester,

2-{2-Chlor-5-[3,6-dihydro-2,6-dioxo-3-methyl-4-trifluormethyl-1(2H)-pyrimidinyl]-benzoyloxy}-2-methyl-propionsäure-allylester,

2-{2-Chlor-5-[3,6-dihydro-2,6-dioxo-3-methyl-4-trifluormethyl-1(2H)-pyrimidinyl]-benzoyloxy}-2-methyl-propionsäure-cyclopentylester,

2-{2-Chlor-5-[3,6-dihydro-2,6-dioxo-3-methyl-4-trifluormethyl-1(2H)-pyrimidinyl]-benzoyloxy}-propionsäure-äthylester,

2-{2-Chlor-5-[3,6-dihydro-2,6-dioxo-3-methyl-4-trifluormethyl-1(2H)-pyrimidinyl]-benzoyloxy}-2-methyl-propionsäure-äthylester,

2-Chlor-5-[3,6-dihydro-2,6-dioxo-3-methyl-4-trifluormethyl-1(2H)-pyrimidinyl]-benzoesäure-(1-acetyläthyl)ester und

3-{2-Chlor-5-[3,6-dihydro-2,6-dioxo-3-methyl-4-trifluormethyl-1(2H)-pyrimidinyl]-benzoyloxy}-butansäure-äthylester.

**8.** Unkrautbekämpfungsmittel, dadurch gekennzeichnet, dass es eine wirksame Menge mindestens einer Verbindung der allgemeinen Formel

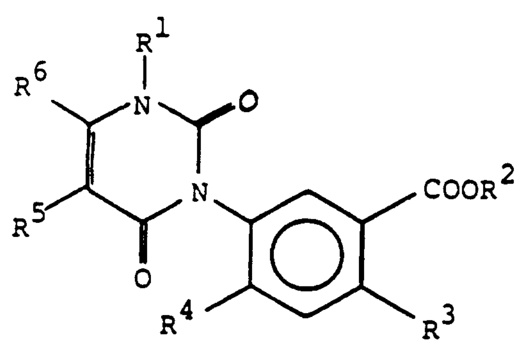

I

worin

| | |
|---|---|
| $R^1$ | Wasserstoff, $C_{1-4}$-Alkyl, $C_{3\ \text{oder}\ 4}$-Alkenyl, $C_{3\ \text{oder}\ 4}$-Alkinyl oder $C_{1-4}$-Halogenalkyl, |
| $R^2$ | $C_{1-6}$-Alkylsulfonyl-$C_{1-4}$-alkyl, Di($C_{1-6}$-alkyl)phosphono-$C_{1-4}$-alkyl, Tri($C_{1-6}$-alkyl)-silyl-$C_{1-4}$-alkyl, $C_{2-7}$-Alkanoyl-$C_{1-4}$-alkyl, Carboxy-$C_{1-4}$-alkyl, ($C_{1-6}$-Alkoxy)-carbonyl-$C_{1-4}$-alkyl, {[$C_{3-9}$-($\alpha$-Alkylalkyliden)]iminooxy -($C_{1-6}$-alkoxy)carbonyl}-$C_{1-4}$-alkyl, ($C_{3-6}$-Alkenyloxy)carbonyl-$C_{1-4}$-alkyl, ($C_{3-6}$-Alkinyloxy)carbonyl-$C_{1-4}$-alkyl, ($C_{3-8}$-Cycloalkyloxy)carbonyl-$C_{1-4}$-alkyl, Phenoxy-$C_{1-4}$-alkyl, $C_{3-6}$-Alkenyloxy-$C_{1-4}$-alkyl, $C_{3-8}$-Cycloalkenyloxy-$C_{1-4}$-alkyl, $C_{5-8}$-Cycloalkenyl, $C_{6-8}$-Bicycloalkyl, $C_{6-8}$-Bicycloalkenyl, $C_{2-7}$-Cyanoalkyl oder $C_{3-6}$-Alkinyloxy-$C_{1-4}$-alkyl, |
| $R^3$ | Halogen oder Cyano, |
| $R^4$ | Wasserstoff oder Halogen, |
| $R^5$ | Wasserstoff, Fluor oder $C_{1-4}$-Alkyl und |
| $R^6$ | $C_{1-4}$-Alkyl oder $C_{1-4}$-Halogenalkyl bedeuten, oder |
| $R^5$ und $R^6$ | zusammen Tri- oder Tetramethylen bilden, |

oder eines Enoläthers einer solchen Verbindung I, in der $R^1$ von Wasserstoff oder $C_{1-4}$-Halogenalkyl verschieden ist, oder eines Salzes einer solchen Verbindung I, in der $R^1$ Wasserstoff bedeutet, sowie Formulierungshilfsstoffe enthält.

**9.** Unkrautbekämpfungsmittel nach Anspruch 8, dadurch gekennzeichnet, dass $R^2$ der Formel I verschieden von Carboxy-$C_{1-4}$-alkyl ist, $R^5$ Wasserstoff, Fluor oder $C_{1-4}$-Alkyl bedeutet und $R^6$ $C_{1-4}$-Alkyl oder $C_{1-4}$-Halogenalkyl bedeutet.

**10.** Unkrautbekämpfungsmittel nach Anspruch 9, dadurch gekennzeichnet, dass es eine wirksame Menge von 2-{2-Chlor-5-[3,6-dihydro-3,4-dimethyl-2,6-dioxo-1(2H)-pyrimidinyl]-4-fluorbenzoyloxy}-2-methyl-propionsäure-äthylester sowie Formulierungshilfsstoffe enthält.

**11.** Unkrautbekämpfungsmittel nach Anspruch 8, dadurch gekennzeichnet, dass es eine wirksame Menge mindestens einer aus der Gruppe
2-{2-Chlor-5-[3,6-dihydro-3,4-dimethyl-2,6-dioxo-1(2H)-pyrimidinyl]-4-fluorbenzoyloxy}-2-methyl-propionsäure-methylester,
2-{2-Chlor-5-[3,6-dihydro-3,4-dimethyl-2,6-dioxo-1(2H)-pyrimidinyl]-4-fluorbenzoyloxy}-2-methyl-propionsäure-(tert.butyl)ester,
2-Aethyl-2-{2-chlor-5-[3,6-dihydro-3,4-dimethyl-2,6-dioxo-1(2H)-pyrimidinyl]-4-fluorbenzoyloxy}-propionsäure-äthylester,
2-{2-Chlor-5-[3,6-dihydro-2,6-dioxo-3-methyl-4-trifluormethyl-1(2H)-pyrimidinyl]-benzoyloxy}-2-methyl-propionsäure-methylester,
2-{2-Chlor-5-[3,6-dihydro-2,6-dioxo-3-methyl-4-trifluormethyl-1(2H)-pyrimidinyl]-benzoyloxy}-propionsäure-isopropylester,
2-Aethyl-2-{2-chlor-5-[3,6-dihydro-2,6-dioxo-3-methyl-4-trifluormethyl-1(2H)-pyrimidinyl]-benzoyloxy}-propionsäure-äthylester,
2-{2-Chlor-5-[3,6-dihydro-2,6-dioxo-3-methyl-4-trifluormethyl-1(2H)-pyrimidinyl]-benzoyloxy}-2-methyl-propionsäure-(tert.butyl)ester,
2-{2-Chlor-5-[3,6-dihydro-2,6-dioxo-3-methyl-4-trifluormethyl-1(2H)-pyrimidinyl]-benzoyloxy}-2-methyl-propionsäure-allylester,
2-{2-Chlor-5-[3,6-dihydro-2,6-dioxo-3-methyl-4-trifluormethyl-1(2H)-pyrimidinyl]-benzoyloxy}-2-methyl-propionsäure-cyclopentylester,
2-{2-Chlor-5-[3,6-dihydro-2,6-dioxo-3-methyl-4-trifluormethyl-1(2H)-pyrimidinyl]-benzoyloxy}-propionsäure-äthylester,
2-{2-Chlor-5-[3,6-dihydro-2,6-dioxo-3-methyl-4-trifluormethyl-1(2H)-pyrimidinyl]-benzoyloxy}-2-methyl-propionsäure-äthylester,
2-Chlor-5-[3,6-dihydro-2,6-dioxo-3-methyl-4-trifluormethyl-1(2H)-pyrimidinyl]-benzoesäure-(1-acetyläthyl)ester und
3-{2-Chlor-5-[3,6-dihydro-2,6-dioxo-3-methyl-4-trifluormethyl-1(2H)-pyrimidinyl]-benzoyloxy}-butansäure-äthylester
ausgewählten Verbindung sowie Formulierungshilfsstoffe enthält.

**12.** Verfahren zur Herstellung von Verbindungen der allgemeinen Formel

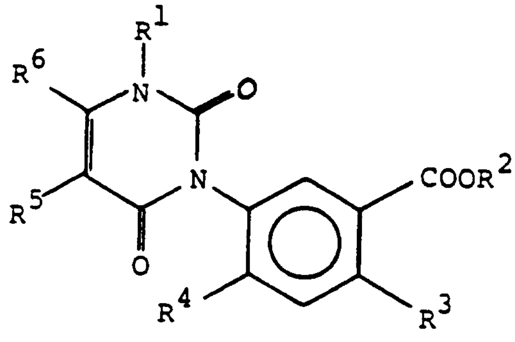

I

worin

| | |
|---|---|
| $R^1$ | Wasserstoff, $C_{1-4}$-Alkyl, $C_{3 \text{ oder } 4}$-Alkenyl, $C_{3 \text{ oder } 4}$-Alkinyl oder $C_{1-4}$-Halogenalkyl, |
| $R^2$ | $C_{1-6}$-Alkylsulfonyl-$C_{1-4}$-alkyl, Di($C_{1-6}$-alkyl)phosphono-$C_{1-4}$-alkyl, Tri($C_{1-6}$-alkyl)- |

silyl-$C_{1-4}$-alkyl, $C_{2-7}$-Alkanoyl-$C_{1-4}$-alkyl, Carboxy-$C_{1-4}$-alkyl, ($C_{1-6}$-Alkoxy)-carbonyl-$C_{1-4}$-alkyl, {[$C_{3-9}$-(α-Alkylalkyliden)]iminooxy -($C_{1-6}$-alkoxy)carbonyl}-$C_{1-4}$-alkyl, ($C_{3-6}$-Alkenyloxy)carbonyl-$C_{1-4}$-alkyl, ($C_{3-6}$-Alkinyloxy)carbonyl-$C_{1-4}$-alkyl, ($C_{3-8}$-Cycloalkyloxy)carbonyl-$C_{1-4}$-alkyl, Phenoxy-$C_{1-4}$-alkyl, $C_{3-6}$-Alkenyloxy-$C_{1-4}$-alkyl, $C_{3-8}$-Cycloalkenyloxy-$C_{1-4}$-alkyl, $C_{5-8}$-Cycloalkenyl, $C_{6-8}$-Bicycloalkyl, $C_{6-8}$-Bicycloalkenyl, $C_{2-7}$-Cyanoalkyl oder $C_{3-6}$-Alkinyloxy-$C_{1-4}$-alkyl,

$R^3$ Halogen oder Cyano,

$R^4$ Wasserstoff oder Halogen,

$R^5$ Wasserstoff, Fluor oder $C_{1-4}$-Alkyl und

$R^6$ $C_{1-4}$-Alkyl oder $C_{1-4}$-Halogenalkyl bedeuten, oder

$R^5$ und $R^6$ zusammen Tri- oder Tetramethylen bilden,

und von den entsprechenden Enoläthern derjenigen Verbindungen der Formel I, in denen $R^1$ von Wasserstoff oder $C_{1-4}$-Halogenalkyl verschieden ist, sowie von Salzen derjenigen Verbindungen der Formel I, in denen $R^1$ Wasserstoff bedeutet, dadurch gekennzeichnet, dass man

a) zwecks Herstellung derjenigen Verbindungen der Formel I, in denen $R^1$ Wasserstoff bedeutet, sowie gewünschtenfalls von Metallsalzen dieser Verbindungen, eine Verbindung der allgemeinen Formel

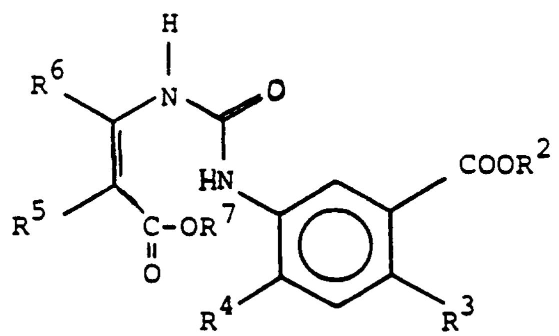

II

worin $R^2$, $R^3$, $R^4$, $R^5$ und $R^6$ die oben angegebenen Bedeutungen besitzen und $R^7$ nieder Alkyl bedeutet,

einer Cyclisierung unter basischen Bedingungen unterwirft und gewünschtenfalls ein allfällig erhaltenes Metallsalz des Uracilderivats der Formel I durch Behandlung mit einer Säure in die saure Form ($R^1$ = Wasserstoff) überführt,

b) zwecks Herstellung derjenigen Verbindungen der Formel I, in denen $R^1$ Wasserstoff bedeutet und $R^6$ verschieden von $C_{1-4}$-Halogenalkyl ist, sowie gewunschtenfalls von Metallsalzen dieser Verbindungen, eine Verbindung der allgemeinen Formel

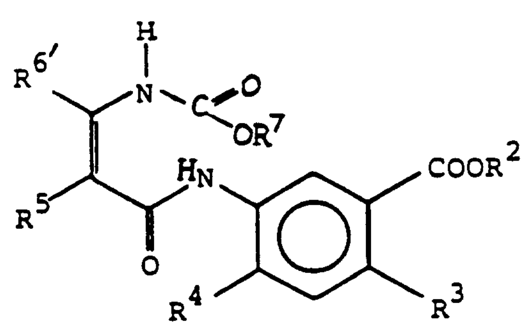

III

worin $R^2$, $R^3$, $R^4$, $R^5$ und $R^7$ die oben angegebenen Bedeutungen besitzen und $R^{6'}$ $C_{1-4}$-Alkyl oder zusammen mit $R^5$ Tri- oder Tetramethylen bedeutet,

einer Cyclisierung unter basischen Bedingungen unterwirft und gewünschtenfalls ein allfällig erhaltenes Metallsalz des Uracilderivats der Formel I durch Behandlung mit einer Säure in die saure Form ($R^1$ = Wasserstoff) überführt,

c) zwecks Herstellung derjenigen Verbindungen der Formel I, in denen $R^1$ $C_{1-4}$-Alkyl, $C_{3\ oder\ 4}$-Alkenyl, $C_{3\ oder\ 4}$-Alkinyl oder $C_{1-4}$-Halogenalkyl bedeutet, ein Uracilderivat der allgemeinen Formel

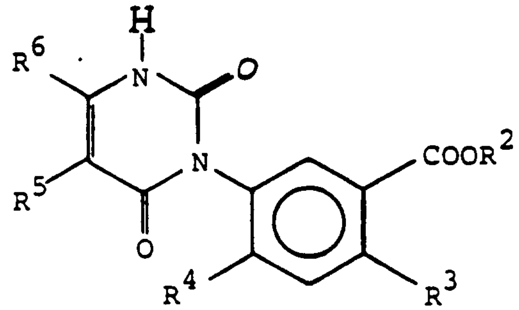

I′

worin $R^2$, $R^3$, $R^4$, $R^5$ und $R^6$ die oben angegebenen Bedeutungen besitzen,
einer Alkylierung mit einem entsprechenden, eine $C_{1-4}$-Alkyl-, $C_{3\ oder\ 4}$-Alkenyl-, $C_{3\ oder\ 4}$-Alkinyl- oder $C_{1-4}$-Halogenalkylgruppe enthaltenden Alkylierungsmittel unterwirft,
d) zwecks Herstellung derjenigen Verbindungen der Formel I, in denen $R^1$ verschieden von Wasserstoff ist, und der entsprechenden Enoläther eine Benzoesäure der allgemeinen Formel

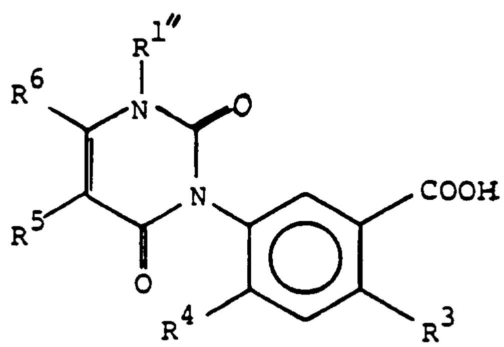

IV

worin $R^{1''}$ $C_{1-4}$-Alkyl, $C_{3\ oder\ 4}$-Alkenyl, $C_{3\ oder\ 4}$-Alkinyl oder $C_{1-4}$-Halogenalkyl bedeutet und $R^3$, $R^4$, $R^5$ und $R^6$ die oben angegebenen Bedeutungen besitzen,
oder den entsprechenden Enoläther, wobei die Benzoesäure bzw. deren Enoläther in Form eines reaktionsfähigen Derivats vorliegen kann, mit einer Hydroxyverbindung der allgemeinen Formel

$HO-R^2$ V

worin $R^2$ die oben angegebene Bedeutung besitzt,
oder mit einem reaktionsfähigen Derivat dieser Hydroxyverbindung verestert,
e) zwecks Herstellung derjenigen Verbindungen der Formel I, in denen $R^1$ verschieden von Wasserstoff ist, einen Benzoesäureester der allgemeinen Formel

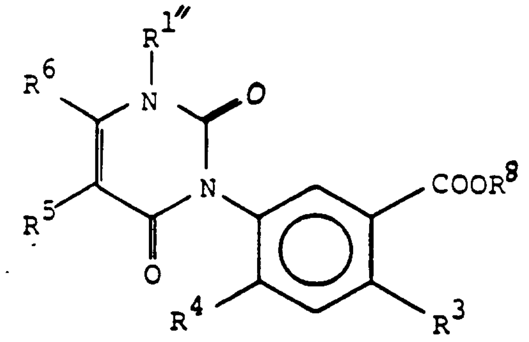

VI

worin $R^{1''}$, $R^3$, $R^4$, $R^5$ und $R^6$ die oben angegebenen Bedeutungen besitzen und $R^8$ $C_{1-6}$-Alkyl, $C_{2-4}$-Alkenyl, $C_{2-4}$-Alkinyl oder $C_{2-6}$-Alkoxyalkyl bedeutet,
einer Umesterungsreaktion mit einer Hydroxyverbindung der oben angegebenen Formel V unterwirft, wobei das Reagens V höher siedend ist als das Alkanol, Alkenol bzw. Alkinol $R^8OH$,
f) zwecks Herstellung derjenigen Verbindungen der Formel I, in denen $R^3$ Cyano bedeutet, und der entsprechenden Enoläther einen 2-Halogen-benzoesäureester der allgemeinen Formel

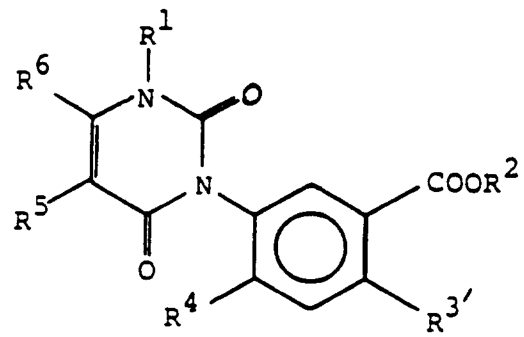

I″

worin $R^{3'}$ Halogen bedeutet und $R^1$, $R^2$, $R^4$, $R^5$ und $R^6$ die oben angegebenen Bedeutungen besitzen,
oder den entsprechenden Enoläther mit einem Metallcyanid behandelt,
g) zwecks Herstellung der Enoläther der Verbindungen der Formel I, ein Uracilderivat der allgemeinen Formel

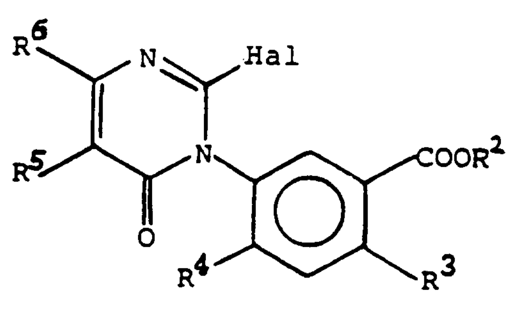

VII

worin $R^2$, $R^3$, $R^4$, $R^5$ und $R^6$ die oben angegebenen Bedeutungen besitzen und Hal Chlor oder Brom bedeutet,
mit einem Alkanol, Alkenol oder Alkinol $R^{1'}OH$ in Gegenwart einer organischen Base oder mit dem entsprechenden Alkoholat, Alkenolat bzw. Alkinolat der allgemeinen Formel

$R^{1'}O^{\ominus}M^{\oplus}$ VIII

worin $R^{1'}$ die oben angegebene Bedeutung besitzt und
$M^{\oplus}$ ein Aequivalent eines Metallions bedeutet,
behandelt,
und gewünschtenfalls eine so erhaltene Verbindung der Formel I, in der $R^1$ Wasserstoff bedeutet, in ein Salz überführt.

**13.** Verfahren nach Anspruch 12, dadurch gekennzeichnet, dass $R^2$ verschieden von Carboxy-$C_{1-4}$-alkyl ist, $R^5$ Wasserstoff, Fluor oder $C_{1-4}$-Alkyl bedeutet und $R^6$ $C_{1-4}$-Alkyl oder $C_{1-4}$-Halogenalkyl bedeutet, und dass die Verbindungen der Formel I und deren Enoläther und Salze durch die Verfahrensvariante a), c), d), e) oder g) und gegebenenfalls durch die Ueberführung in ein Salz hergestellt werden.

**14.** Verfahren nach Anspruch 12, dadurch gekennzeichnet, dass man eine Verbindung gemäss einem der Ansprüche 1 bis 7 herstellt.

**15.** Verfahren zur Bekämpfung von Unkräutern, dadurch gekennzeichnet, dass man das gegen Unkräuter zu schützende Gut und/oder die Unkräuter mit einer wirksamen Menge einer Verbindung gemäss einem der Ansprüche 1, 4, 5 und 7 bzw. eines Mittels gemäss Anspruch 8 oder 11 behandelt.

**16.** Verfahren zur Bekämpfung von Unkräutern, dadurch gekennzeichnet, dass man das gegen Unkräuter zu schützende Gut und/oder die Unkräuter mit einer wirksamen Menge einer Verbindung gemäss einem der Ansprüche 2, 3 und 6 bzw. eines Mittels gemäss Anspruch 9 oder 10 behandelt.

**17.** Verwendung einer Verbindung gemäss einem der Ansprüche 1, 4, 5 und 7 bzw. eines Mittels gemäss Anspruch 8 oder 11 zur Bekämpfung von Unkräutern.

**18.** Verwendung einer Verbindung gemäss einem der Ansprüche 2, 3 und 6 bzw. eines Mittels gemäss Anspruch 9 oder 10 zur Bekämpfung von Unkräutern.

## Claims

**1.** Compounds of the general formula

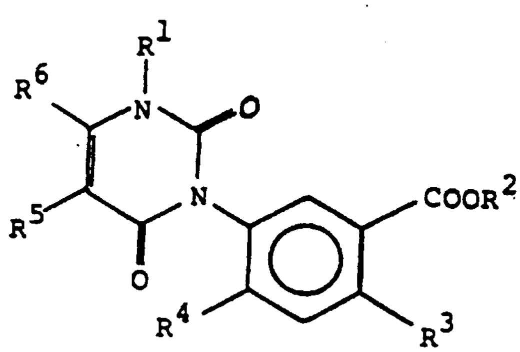

I

wherein

$R^1$ signifies hydrogen, $C_{1-4}$alkyl, $C_{3\ or\ 4}$alkenyl, $C_{3\ or\ 4}$alkynyl or $C_{1-4}$haloalkyl,

$R^2$ signifies $C_{1-6}$alkylsulfonyl-$C_{1-4}$alkyl, di($C_{1-6}$alkyl)phosphono-$C_{1-4}$alkyl, tri-($C_{1-6}$alkyl)silyl-$C_{1-4}$alkyl, $C_{2-7}$alkanoyl-$C_{1-4}$alkyl, carboxy-$C_{1-4}$alkyl, ($C_{1-6}$alkoxy)-carbonyl-$C_{1-4}$alkyl, {[$C_{3-9}$($\alpha$-alkylalkylidene)]iminooxy ($C_{1-6}$alkoxy)carbonyl}-$C_{1-4}$alkyl, ($C_{3-6}$alkenyloxy)carbonyl-$C_{1-4}$alkyl, ($C_{3-6}$alkynyloxy)carbonyl-$C_{1-4}$alkyl, ($C_{3-8}$cycloalkyloxy)carbonyl-$C_{1-4}$alkyl, phenoxy-$C_{1-4}$alkyl, $C_{3-6}$alkenyloxy-$C_{1-4}$alkyl, $C_{3-8}$cycloalkenyloxy-$C_{1-4}$alkyl, $C_{5-8}$cycloalkenyl, $C_{6-8}$bicycloalkyl, $C_{6-8}$bicycloal-

kenyl, $C_{2-7}$cyanoalkyl or $C_{3-6}$alkynyloxy-$C_{1-4}$alkyl,

$R^3$ signifies halogen or cyano,

$R^4$ signifies hydrogen or halogen,

$R^5$ signifies hydrogen, fluorine or $C_{1-4}$alkyl and

$R^6$ signifies $C_{1-4}$alkyl or $C_{1-4}$haloalkyl or

$R^5$ and $R^6$ together form trimethylene or tetramethylene,

and the corresponding enol ethers of those compounds of formula I in which $R^1$ is other than hydrogen or $C_{1-4}$haloalkyl as well as salts of those compounds of formula I in which $R^1$ signifies hydrogen.

**2.** Compounds according to claim 1, wherein $R^2$ is other than carboxy-$C_{1-4}$alkyl, $R^5$ signifies hydrogen, fluorine or $C_{1-4}$alkyl and $R^6$ signifies $C_{1-4}$alkyl or $C_{1-4}$haloalkyl, as well as their enol ethers and salts.

**3.** Compounds according to claim 1 or 2, wherein $R^1$ signifies methyl, $R^3$ signifies chlorine or bromine, $R^4$ signifies hydrogen or fluorine, $R^5$ signifies hydrogen, fluorine or methyl and $R^6$ signifies methyl, trifluoromethyl or pentafluoromethyl.

**4.** Compounds according to claim 1, wherein $R^3$ signifies cyano.

**5.** Compounds according to any one of claims 1 to 4, wherein $R^2$ signifies $C_{2-7}$alkanoyl-$C_{1-4}$alkyl, ($C_{1-6}$alkoxy)carbonyl-$C_{1-4}$alkyl, {[$C_{3-9}$($\alpha$-alkylalkylidene)]iminooxy($C_{1-6}$alkoxy)carbonyl}-$C_{1-4}$alkyl, ($C_{3-6}$alkenyloxy)carbonyl-$C_{1-4}$alkyl, ($C_{3-6}$alkynyloxy)carbonyl-$C_{1-4}$alkyl or ($C_{3-8}$cycloalkyloxy)-carbonyl-$C_{1-4}$alkyl.

**6.** A compound according to claim 2, ethyl 2-{2-chloro-5-[3,6-dihydro-3,4-dimethyl-2,6-dioxo-1(2H)-pyrimidinyl]-4-fluorobenzoyloxy}-2-methyl-propionate.

**7.** A compound according to claim 1, selected from:

methyl 2-{2-chloro-5-[3,6-dihydro-3,4-dimethyl-2,6-dioxo-1(2H)-pyrimidinyl]-4-fluorobenzoyloxy}-2-methylpropionate,

tert-butyl 2-{2-chloro-5-[3,6-dihydro-3,4-dimethyl-2,6-dioxol-1(2H)-pyrimidinyl]-4-fluorobenzoyloxy}-2-methylpropionate,

ethyl 2-ethyl-2-{2-chloro-5-[3,6-dihydro-3,4-dimethyl-2,6-dioxo-1(2H)-pyrimidinyl]-4-fluorobenzoyloxy}propionate,

methyl 2-{2-chloro-5-[3,6-dihydro-2,6-dioxo-3-methyl-4-trifluoromethyl-1(2H)-pyrimidinyl]benzoyloxy}-2-methylpropionate,

isopropyl 2-{2-chloro-5-[3,6-dihydro-2,6-dioxo-3-methyl-4-trifluoromethyl-1(2H)-pyrimidinyl]-benzoyloxy}propionate,

ethyl 2-ethyl-2-{2-chloro-5-[3,6-dihydro-2,6-dioxo-3-methyl-4-trifluoromethyl-1(2H)-pyrimidinyl]-benzoyloxy}propionate,

tert-butyl 2-{2-chloro-5-[3,6-dihydro-2,6-dioxo-3-methyl-4-trifluoromethyl-1(2H)-pyrimidinyl]benzoyloxy}-2-methylpropionate,

allyl 2-{2-chloro-5-[3,6-dihydro-2,6-dioxo-3-methyl-4-trifluoromethyl-1(2H)-pyrimidinyl]benzoyloxy}-2-methylpropionate,

cyclopentyl 2-{2-chloro-5-[3,6-dihydro-2,6-dioxo-3-methyl-4-trifluoromethyl-1(2H)-pyrimidinyl]-benzoyloxy}-2-methylpropionate,

ethyl 2-{2-chloro-5-[3,6-dihydro-2,6-dioxo-3-methyl-4-trifluoromethyl-1(2H)-pyrimidinyl]-benzoyloxy}propionate,

ethyl 2-{2-chloro-5-[3,6-dihydro-2,6-dioxo-3-methyl-4-trifluoromethyl-1(2H)-pyrimidinyl]benzoyloxy}-2-methylpropionate,

1-acetylethyl 2-chloro-5-[3,6-dihydro-2,6-dioxo-3-methyl-4-trifluoromethyl-1(2H)-pyrimidinyl]benzoate and

ethyl 3-{2-chloro-5-[3,6-dihydro-2,6-dioxo-3-methyl-4-trifluoromethyl-1(2H)-pyrimidinyl]-benzoyloxy}butanoate.

**8.** A weed control composition, which contains an effective amount of at least one compound of the general formula

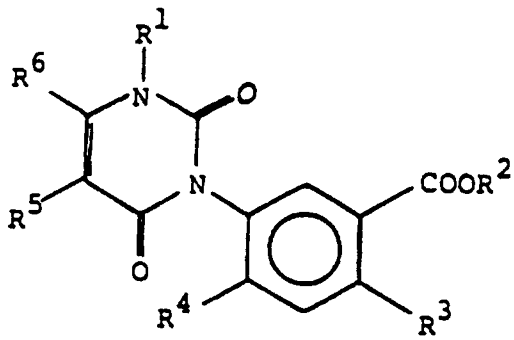 I

wherein

| | |
|---|---|
| $R^1$ | signifies hydrogen, $C_{1-4}$alkyl, $C_{3\ or\ 4}$alkenyl, $C_{3\ or\ 4}$alkynyl or $C_{1-4}$haloalkyl, |
| $R^2$ | signifies $C_{1-6}$alkylsulfonyl-$C_{1-4}$alkyl, di($C_{1-6}$alkyl)phosphono-$C_{1-4}$alkyl, tri-($C_{1-6}$alkyl)silyl-$C_{1-4}$alkyl, $C_{2-7}$alkanoyl-$C_{1-4}$alkyl, carboxy-$C_{1-4}$alkyl, ($C_{1-6}$alkoxy)-carbonyl-$C_{1-4}$alkyl, {[$C_{3-9}$($\alpha$-alkylalkylidene)]iminooxy ($C_{1-6}$alkoxy)carbonyl}-$C_{1-4}$alkyl, ($C_{3-6}$alkenyloxy)carbonyl-$C_{1-4}$alkyl, ($C_{3-6}$alkynyloxy)carbonyl-$C_{1-4}$alkyl, ($C_{3-8}$cycloalkyloxy)carbonyl-$C_{1-4}$alkyl, phenoxy-$C_{1-4}$alkyl, $C_{3-6}$alkenyloxy-$C_{1-4}$alkyl, $C_{3-8}$cycloalkenyloxy-$C_{1-4}$alkyl, $C_{5-8}$cycloalkenyl, $C_{6-8}$bicycloalkyl, $C_{6-8}$bicycloalkenyl, $C_{2-7}$cyanoalkyl or $C_{3-6}$alkynyloxy-$C_{1-4}$alkyl, |
| $R^3$ | signifies halogen or cyano, |
| $R^4$ | signifies hydrogen or halogen, |
| $R^5$ | signifies hydrogen, fluorine or $C_{1-4}$alkyl and |
| $R^6$ | signifies $C_{1-4}$alkyl or $C_{1-4}$haloalkyl or |
| $R^5$ and $R^6$ | together form trimethylene or tetramethylene |

or of an enol ether of such a compound I in which $R^1$ is other than hydrogen or $C_{1-4}$haloalkyl or of a salt of such a compound I in which $R^1$ signifies hydrogen
as well as formulation adjuvants.

**9.** A weed control composition according to claim 8, wherein $R^2$ in formula I is other than carboxy-$C_{1-4}$alkyl, $R^5$ signifies hydrogen, fluorine or $C_{1-4}$alkyl and $R^6$ signifies $C_{1-4}$alkyl or $C_{1-4}$haloalkyl.

**10.** A weed control composition according to claim 9, which contains an effective amount of ethyl 2-{2-chloro-5-[3,6-dihydro-3,4-dimethyl-2,6-dioxo-1(2H)-pyrimidinyl]-4-fluorobenzoyloxy}-2-methylpropionate as well as formulation adjuvants.

**11.** A weed control composition according to claim 8, which contains an effective amount of at least one compound selected from the group
methyl 2-{2-chloro-5-[3,6-dihydro-3,4-dimethyl-2,6-dioxo-1(2H)-pyrimidinyl]-4-fluorobenzoyloxy}-2-methylpropionate,
tert-butyl 2-{2-chloro-5-[3,6-dihydro-3,4-dimethyl-2,6-dioxo-1(2H)-pyrimidinyl]-4-fluorobenzoyloxy}-2-methylpropionate,
ethyl 2-ethyl-2-{2-chloro-5-[3,6-dihydro-3,4-dimethyl-2,6-dioxo-1(2H)-pyrimidinyl]-4-fluorobenzoyloxy}propionate,
methyl 2-{2-chloro-5-[3,6-dihydro-2,6-dioxo-3-methyl-4-trifluoromethyl-1(2H)-pyrimidinyl]benzoyloxy}-2-methylpropionate,
isopropyl 2-{2-chloro-5-[3,6-dihydro-2,6-dioxo-3-methyl-4-trifluoromethyl-1(2H)-pyrimidinyl]-benzoyloxy}propionate,
ethyl 2-ethyl-2-{2-chloro-5-[3,6-dihydro-2,6-dioxo-3-methyl-4-trifluoromethyl-1(2H)-pyrimidinyl]-benzoyloxy}propionate,
tert-butyl 2-{2-chloro-5-[3,6-dihydro-2,6-dioxo-3-methyl-4-trifluoromethyl-1(2H)-pyrimidinyl]benzoyloxy}-2-methylpropionate,
allyl 2-{2-chloro-5-[3,6-dihydro-2,6-dioxo-3-methyl-4-trifluoromethyl-1(2H)-pyrimidinyl]benzoyloxy}-2-methylpropionate,

cyclopentyl 2-{2-chloro-5-[3,6-dihydro-2,6-dioxo-3-methyl-4-trifluoromethyl-1(2H)-pyrimidinyl]-benzoyloxy}-2-methylpropionate,
ethyl 2-{2-chloro-5-[3,6-dihydro-2,6-dioxo-3-methyl-4-trifluoromethyl-1(2H)-pyrimidinyl]-benzoyloxy}propionate,
ethyl 2-{2-chloro-5-[3,6-dihydro-2,6-dioxo-3-methyl-4-trifluoromethyl-1(2H)-pyrimidinyl]benzoyloxy}-2-methylpropionate,
1-acetylethyl 2-chloro-5-[3,6-dihydro-2,6-dioxo-3-methyl-4-trifluoromethyl-1(2H)-pyrimidinyl]benzoate and
ethyl 3-{2-chloro-5-[3,6-dihydro-2,6-dioxo-3-methyl-4-trifluoromethyl-1(2H)-pyrimidinyl]-benzoyloxy}butanoate
as well as formulation adjuvants.

**12.** A process for the manufacture of compounds of the general formula

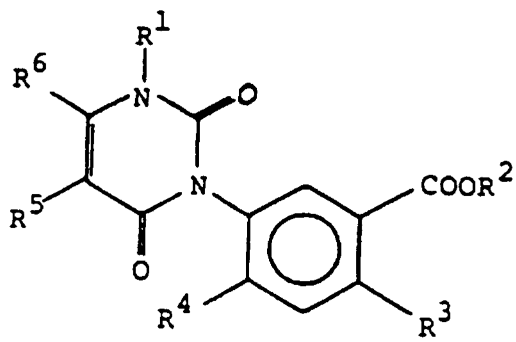

I

wherein

$R^1$ signifies hydrogen, $C_{1-4}$alkyl, $C_{3\ or\ 4}$alkenyl, $C_{3\ or\ 4}$alkynyl or $C_{1-4}$haloalkyl,

$R^2$ signifies $C_{1-6}$alkylsulfonyl-$C_{1-4}$alkyl, di($C_{1-6}$alkyl)phosphono-$C_{1-4}$alkyl, tri-($C_{1-6}$alkyl)silyl-$C_{1-4}$alkyl, $C_{2-7}$alkanoyl-$C_{1-4}$alkyl, carboxy-$C_{1-4}$alkyl, ($C_{1-6}$alkoxy)-carbonyl-$C_{1-4}$alkyl, {[$C_{3-9}$($\alpha$-alkylalkylidene)]iminooxy ($C_{1-6}$alkoxy)carbonyl}-$C_{1-4}$alkyl, ($C_{3-6}$alkenyloxy)carbonyl-$C_{1-4}$alkyl, ($C_{3-6}$alkynyloxy)carbonyl-$C_{1-4}$alkyl, ($C_{3-8}$cycloalkyloxy)carbonyl-$C_{1-4}$alkyl, phenoxy-$C_{1-4}$alkyl, $C_{3-6}$alkenyloxy-$C_{1-4}$alkyl, $C_{3-8}$cycloalkenyloxy-$C_{1-4}$alkyl, $C_{5-8}$cycloalkenyl, $C_{6-8}$bicycloalkyl, $C_{6-8}$bicycloalkenyl, $C_{2-7}$cyanoalkyl or $C_{3-6}$alkynyloxy-$C_{1-4}$alkyl,

$R^3$ signifies halogen or cyano,

$R^4$ signifies hydrogen or halogen,

$R^5$ signifies hydrogen, fluorine or $C_{1-4}$alkyl and

$R^6$ signifies $C_{1-4}$alkyl or $C_{1-4}$haloalkyl or

$R^5$ and $R^6$ together form trimethylene or tetramethylene,

and of the corresponding enol ethers of those compounds of formula I in which $R^1$ is other than hydrogen or $C_{1-4}$haloalkyl as well as of salts of those compounds of formula I in which $R^1$ signifies hydrogen, which process comprises

a) for the manufacture of those compounds of formula I in which $R^1$ signifies hydrogen as well as, if desired, of metal salts of these compounds, subjecting a compound of the general formula

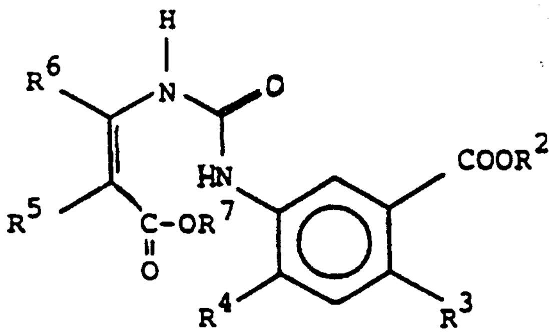

II

wherein $R^2$, $R^3$, $R^4$, $R^5$ and $R^6$ have the significations given above and $R^7$ signifies lower alkyl, to a cyclization under basic conditions and, if desired, converting a metal salt of the uracil derivative of formula I which may be obtained into the acidic form ($R^1$ = hydrogen) by treatment with an acid,
b) for the manufacture of those compounds of formula I in which $R^1$ signifies hydrogen and $R^6$ is other than $C_{1-4}$haloalkyl as well as, if desired, of metal salts of these compounds, subjecting a compound of the general formula

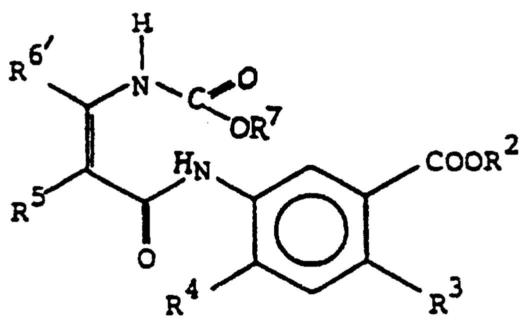

III

wherein $R^2$, $R^3$, $R^4$, $R^5$ and $R^7$ have the significations given above and $R^{6'}$ signifies $C_{1-4}$alkyl or together with $R^5$ signifies trimethylene or tetramethylene,
to a cyclization under basic conditions and, if desired, converting a metal salt of the uracil derivative of formula I which may be obtained into the acidic form ($R^1$ = hydrogen) by treatment with an acid,
c) for the manufacture of those compounds of formula I in which $R^1$ signifies $C_{1-4}$alkyl, $C_{3\ \mathrm{or}\ 4}$alkenyl, $C_{3\ \mathrm{or}\ 4}$alkynyl or $C_{1-4}$haloalkyl, subjecting a uracil derivative of the general formula

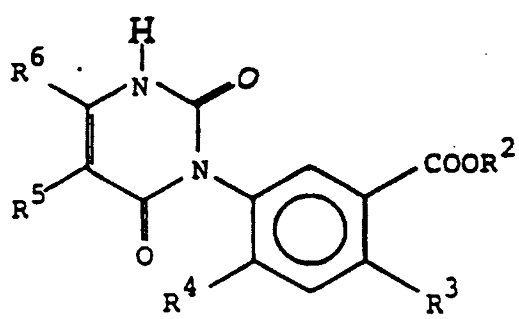

I'

wherein $R^2$, $R^3$, $R^4$, $R^5$ and $R^6$ have the significances given above, to an alkylation with an appropriate alkylating agent comprising a $C_{1-4}$alkyl, $C_{3\ \mathrm{or}\ 4}$alkenyl, $C_{3\ \mathrm{or}\ 4}$alkynyl or $C_{1-4}$haloalkyl group,
d) for the manufacture of those compounds of formula I in which $R^1$ is other than hydrogen and of the corresponding enol ethers, esterifying a benzoic acid of the general formula

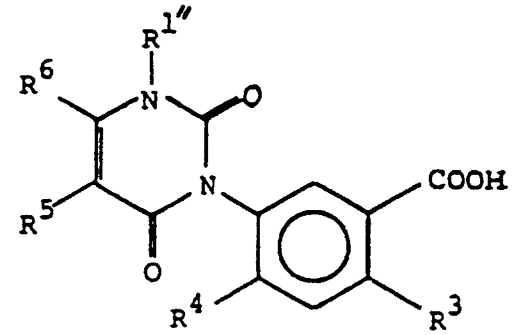

IV

wherein $R^{1''}$ signifies $C_{1-4}$alkyl, $C_{3\ or\ 4}$alkenyl, $C_{3\ or\ 4}$alkynyl or $C_{1-4}$haloalkyl and $R^3$, $R^4$, $R^5$ and $R^6$ have the significations given above, or the corresponding enol ether, where the benzoic acid or its enol ether can be present in the form of a reactive derivative, with a hydroxy compound of the general formula

$HO\text{-}R^2$ V

wherein $R^2$ has the significations given above,
or with a reactive derivative of this hydroxy compound,
e) for the manufacture of those compounds of formula I in which $R^1$ is other than hydrogen, subjecting a benzoic acid ester of the general formula

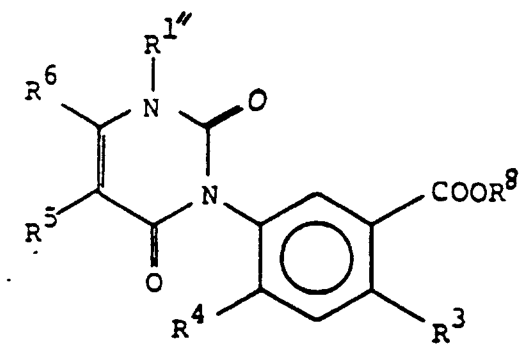

VI

wherein $R^{1''}$, $R^3$, $R^4$, $R^5$ and $R^6$ have the significations given above and $R^8$ signifies $C_{1-6}$alky, $C_{2-4}$alkenyl, $C_{2-4}$alkynyl or $C_{2-6}$alkoxyalkyl, to a transesterification reaction with a hydroxy compound of formula V given above, where the reagent V has a higher boiling point than the alkanol, alkenol or alkynol $R^8OH$,
f) for the manufacture of those compounds of formula I in which $R^3$ signifies cyano and of the corresponding enol ethers, treating a 2-halobenzoate of the general formula

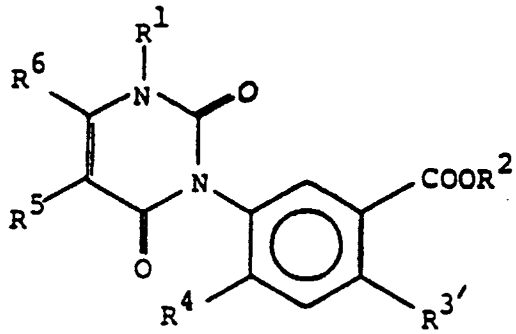

I″

wherein $R^{3'}$ signifies halogen and $R^1$, $R^2$, $R^4$, $R^5$ and $R^6$ have the significations given above or the corresponding enol ether with a metal cyanide,

g) for the manufacture of enol ethers of the compounds of formula I, treating a uracil derivative of the general formula

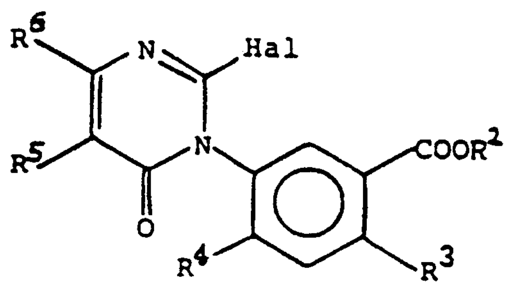

VII

wherein $R^2$, $R^3$, $R^4$, $R^5$ and $R^6$ have the significations given above and Hal signifies chlorine or bromine,

with an alkanol, alkenol or alkynol $R^{1'}OH$ in the presence of an organic base or with the corresponding alcoholate, alkenolate or alkynolate of the general formula

$R^{1'}\ O^{\ominus}M^{\oplus}$ VIII

wherein $R^{1'}$ has the significations given above and $M^{\oplus}$ signifies one equivalent of a metal ion,

and, if desired, converting a thus-obtained compound of formula I in which $R^1$ signifies hydrogen into a salt.

**13.** A process according to claim 12, wherein $R^2$ is other than carboxy-$C_{1-4}$alkyl, $R^5$ signifies hydrogen, fluorine or $C_{1-4}$alkyl and $R^6$ signifies $C_{1-4}$alkyl or $C_{1-4}$haloalkyl and wherein the compounds of formula I and their enol ethers and salts are manufactured by process variant a), c), d), e) or g) and, if desired, by conversion into a salt.

**14.** A process according to claim 12, wherein a compound according to any one of claims 1 to 7 is manufactured.

**15.** A method for the control of weeds, which method comprises treating the material to be protected against weeds and/or the weeds with an effective amount of a compound in accordance with any one of claims 1, 4, 5 and 7 or of a composition in accordance with claims 8 or 11.

**16.** A method for the control of weeds, which method comprises treating the material to be protected against weeds and/or the weeds with an effective amount of a compound in accordance with any one of claims 2, 3 and 6 or of a composition in accordance with claim 9 or 10.

**17.** The use of a compound in accordance with any one of claims 1, 4, 5 and 7 or of a composition in accordance with claim 8 or 11 for the control of weeds.

**18.** The use of a compound in accordance with any one of claims 2, 3 and 6 or of a composition in accordance with claim 9 or 10 for the control of weeds.

## Revendications

**1.** Composés de formule générale

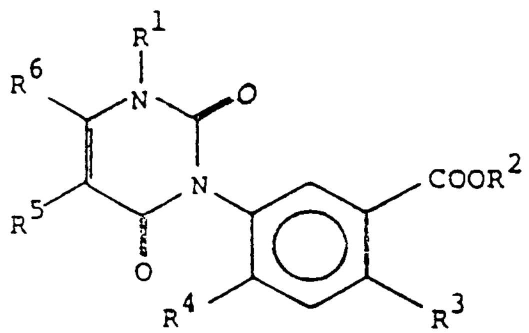

I

dans laquelle

$R^1$ représente un atome d'hydrogène ou un groupe alkyle en $C_{1-4}$, alcényle en $C_{3\ ou\ 4}$, alcynyle en $C_{3\ ou\ 4}$ ouhalogénoalkyle en $C_{1-4}$,

$R^2$ représente un groupe alkyl($C_{1-6}$)sulfonyl-alkyle($C_{1-4}$), dialkyl($C_{1-6}$)phosphono-alkyle-($C_{1-4}$), trialkyl($C_{1-6}$)silyl-alkyle($C_{1-4}$), alcanoyl($C_{2-7}$)-alkyle($C_{1-4}$), carboxy-alkyle-($C_{1-4}$), alcoxy($C_{1-6}$)carbonyl-alkyle($C_{1-4}$), {[α-alkylalkylidène($C_{3-9}$)]iminooxy-alcoxy-($C_{1-6}$)carbonyl}-alkyle($C_{1-4}$), alcényloxy($C_{3-6}$)carbonyl-alkyle($C_{1-4}$), alcynyloxy($C_{3-6}$)-carbonyl-alkyle($C_{1-4}$), cycloalkyloxy($C_{3-8}$)carbonyl-alkyle($C_{1-4}$), phénoxy-alkyle($C_{1-4}$), alcényloxy($C_{3-6}$)-alkyle($C_{1-4}$), cycloalcényloxy($C_{3-8}$)-alkyle($C_{1-4}$), cycloalcényle en $C_{5-8}$, bicycloalkyle en $C_{6-8}$, bicycloalcényle en $C_{6-8}$, cyanoalkyle($C_{2-7}$) ou alcynyloxy($C_{3-6}$)-alkyle($C_{1-4}$),

$R^3$ représente un atome d'halogène ou le groupe cyano,

$R^4$ représente un atome d'hydrogène ou d'halogène,

$R^5$ représente un atome d'hydrogène ou de fluor ou un groupe alkyle en $C_{1-4}$, et

$R^6$ représente un groupe alkyle en $C_{1-4}$ ou halogénoalkyle en $C_{1-4}$, ou

$R^5$ et $R^6$ forment ensemble un groupe tri- ou tétraméthylène,

et énoléthers correspondants des composés de formule I dans lesquels $R^1$ est différent d'un atome d'hydrogène ou d'un groupe halogénoalkyle en $C_{1-4}$, et sels des composés de formule I dans lesquels $R^1$ représente un atome d'hydrogène.

**2.** Composés selon la revendication 1, dans lesquels $R^2$ est différent d'un groupe carboxy-alkyle($C_{1-4}$), $R^5$ représente un atome d'hydrogène ou de fluor ou un groupe alkyle en $C_{1-4}$, et $R^6$ représente un groupe alkyle en $C_{1-4}$ ou halogénoalkyle en $C_{1-4}$, et énoléthers et sels de ceux-ci.

**3.** Composés selon la revendication 1 ou 2, dans lesquels $R^2$ représente le groupe méthyle, $R^3$ représente un atome de chlore ou de brome, $R^4$ représente un atome d'hydrogène ou de fluor, $R^5$ représente un atome d'hydrogène ou de fluor ou le groupe méthyle, et $R^6$ représente le groupe méthyle, trifluorométhyle ou pentafluorométhyle.

**4.** Composés selon la revendication 1, dans lesquels $R^3$ représente le groupe cyano.

**5.** Composés selon l'une des revendications 1 à 4, dans lesquels $R^2$ représente un groupe alcanoyl-($C_{2-7}$)-alkyle($C_{1-4}$), alcoxy($C_{1-6}$)carbonyl-alkyle($C_{1-4}$), {[α-alkylalkylidène($C_{3-9}$)]iminooxy-alcoxy-($C_{1-6}$)carbonyl}-alkyle($C_{1-4}$), alcényloxy($C_{3-6}$)carbonyl-alkyle($C_{1-4}$), alcynyloxy($C_{3-6}$)carbonyl-alkyle-

($C_{1-4}$) ou cycloalkyloxy($C_{3-8}$)carbonyl-alkyle($C_{1-4}$).

6. Composé selon la revendication 2, qui est le 2-{2-chloro-5-[3,6-dihydro-3,4-diméthyl-2,6-dioxo-1(2H)-pyrimidinyl]-4-fluorobenzoyloxy}-2-méthyl-propionate d'éthyle.

7. Composé selon la revendication 1, choisi parmi
le 2-{2-chloro-5-[3,6-dihydro-3,4-diméthyl-2,6-dioxo-1(2H)-pyrimidinyl]-4-fluorobenzoyloxy}-2-méthyl-propionate de méthyle,
le 2-{2-chloro-5-[3,6-dihydro-3,4-diméthyl-2,6-dioxo-1(2H)-pyrimidinyl]-4-fluorobenzoyloxy}-2-méthyl-propionate de tert-butyle,
le 2-éthyl-2-{2-chloro-5-[3,6-dihydro-3,4-diméthyl-2,6-dioxo-1(2H)-pyrimidinyl]-4-fluorobenzoyloxy}-propionate d'éthyle,
le 2-{2-chloro-5-[3,6-dihydro-2,6-dioxo-3-méthyl-4-trifluorométhyl-1(2H)-pyrimidinyl]-benzoyloxy}-2-méthyl-propionate de méthyle,
le 2-{2-chloro-5-[3,6-dihydro-2,6-dioxo-3-méthyl-4-trifluorométhyl-1(2H)-pyrimidinyl]-benzoyloxy}-propionate d'isopropyle,
le 2-éthyl-2-{2-chloro-5-[3,6-dihydro-2,6-dioxo-3-méthyl-4-trifluorométhyl-1(2H)-pyrimidinyl]-benzoyloxy}-propionate d'éthyle,
le 2-{2-chloro-5-[3,6-dihydro-2,6-dioxo-3-méthyl-4-trifluorométhyl-1(2H)-pyrimidinyl]-benzoyloxy}-2-méthyl-propionate de tert-butyle,
le 2-{2-chloro-5-[3,6-dihydro-2,6-dioxo-3-méthyl-4-trifluorométhyl-1(2H)-pyrimidinyl]-benzoyloxy}-2-méthyl-propionate d'allyle,
le 2-{2-chloro-5-[3,6-dihydro-2,6-dioxo-3-méthyl-4-trifluorométhyl-1(2H)-pyrimidinyl]-benzoyloxy}-2-méthyl-propionate de cyclopentyle,
le 2-{2-chloro-5-[3,6-dihydro-2,6-dioxo-3-méthyl-4-trifluorométhyl-1(2H)-pyrimidinyl]-benzoyloxy}-propionate d'éthyle,
le 2-{2-chloro-5-[3,6-dihydro-2,6-dioxo-3-méthyl-4-trifluorométhyl-1(2H)-pyrimidinyl]-benzoyloxy}-2-méthyl-propionate d'éthyle,
le 2-chloro-5-[3,6-dihydro-2,6-dioxo-3-méthyl-4-trifluorométhyl-1(2H)-pyrimidinyl]-benzoate de 1-acétyléthyle et
le 3-{2-chloro-5-[3,6-dihydro-2,6-dioxo-3-méthyl-4-trifluorométhyl-1(2H)-pyrimidinyl]-benzoyloxy}-butanoate d'éthyle.

8. Composition herbicide, caractérisée en ce qu'elle contient une quantité efficace d'au moins un composé de formule générale

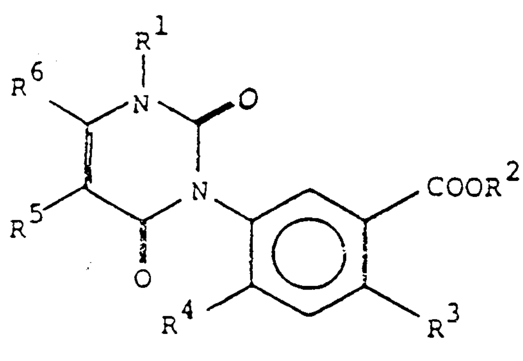

I

dans laquelle

$R^1$ représente un atome d'hydrogène ou un groupe alkyle en $C_{1-4}$, alcényle en $C_{3\ ou\ 4}$, alcynyle en $C_{3\ ou\ 4}$ ouhalogénoalkyle en $C_{1-4}$,

$R^2$ représente un groupe alkyl($C_{1-6}$)sulfonyl-alkyle($C_{1-4}$), dialkyl($C_{1-6}$)phosphono-alkyle-($C_{1-4}$), trialkyl($C_{1-6}$)silyl-alkyle($C_{1-4}$), alcanoyl($C_{2-7}$)-alkyle($C_{1-4}$), carboxy-alkyle-($C_{1-4}$), alcoxy($C_{1-6}$)carbonyl-alkyle($C_{1-4}$), {[α-alkylalkylidène($C_{3-9}$)]iminooxy-alcoxy-($C_{1-6}$)carbonyl}-alkyle($C_{1-4}$), alcényloxy($C_{3-6}$)carbonyl-alkyle($C_{1-4}$), alcynyloxy($C_{3-6}$)-carbonyl-alkyle($C_{1-4}$), cycloalkyloxy($C_{3-8}$)carbonyl-alkyle($C_{1-4}$), phénoxy-alkyle($C_{1-4}$), alcényloxy($C_{3-6}$)-alkyle($C_{1-4}$), cycloalcényloxy($C_{3-8}$)-alkyle($C_{1-4}$), cycloalcényle en

$C_{5-8}$,bicycloalkyle en $C_{6-8}$, bicycloalcényle en $C_{6-8}$, cyanoalkyle($C_{2-7}$) ou alcynyloxy-($C_{3-6}$)-alkyle($C_{1-4}$),

$R^3$ représente un atome d'halogène ou le groupe cyano,

$R^4$ représente un atome d'hydrogène ou d'halogène,

$R^5$ représente un atome d'hydrogène ou de fluor ou un groupe alkyle en $C_{1-4}$, et

$R^6$ représente un groupe alkyle en $C_{1-4}$ ou halogénoalkyle en $C_{1-4}$, ou

$R^5$ et $R^6$ forment ensemble un groupe tri- ou tétraméthylène,

ou d'un énoléther d'un tel composé I dans lequel $R^1$ est différent d'un atome d'hydrogène ou d'un groupe halogénoalkyle en $C_{1-4}$, ou d'un sel d'un tel composé I dans lequel $R^1$ représente un atome d'hydrogène,

ainsi que des adjuvants de formulation.

**9.** Composition herbicide selon la revendication 8, caractérisée en ce que $R^2$ de la formule I est différent d'un groupe carboxyalkyle($C_{1-4}$), $R^5$ représente un atome d'hydrogène ou de fluor ou un groupe alkyle en $C_{1-4}$, et $R^6$ représenteun groupe alkyle en $C_{1-4}$ ou halogénoalkyle en $C_{1-4}$.

**10.** Composition herbicide selon la revendication 9, caractérisée en ce qu'elle contient une quantité efficace de 2-{2-chloro-5-[3,6-dihydro-3,4-diméthyl-2,6-dioxo-1(2H)-pyrimidinyl]-4-fluorobenzoyloxy}-2-méthyl-propionate d'éthyle ainsi que des adjuvants de formulation.

**11.** Composition herbicide selon la revendication 8, caractérisée en ce qu'elle contient une quantité efficace d'au moins un composé choisi parmi

le 2-{2-chloro-5-[3,6-dihydro-3,4-diméthyl-2,6-dioxo-1(2H)-pyrimidinyl]-4-fluorobenzoyloxy}-2-méthyl-propionate de méthyle,

le 2-{2-chloro-5-[3,6-dihydro-3,4-diméthyl-2,6-dioxo-1(2H)-pyrimidinyl]-4-fluorobenzoyloxy}-2-méthyl-propionate de tert-butyle,

le 2-éthyl-2-{2-chloro-5-[3,6-dihydro-3,4-diméthyl-2,6-dioxo-1(2H)-pyrimidinyl]-4-fluorobenzoyloxy}-propionate d'éthyle,

le 2-{2-chloro-5-[3,6-dihydro-2,6-dioxo-3-méthyl-4-trifluorométhyl-1(2H)-pyrimidinyl]-benzoyloxy}-2-méthyl-propionate de méthyle,

le 2-{2-chloro-5-[3,6-dihydro-2,6-dioxo-3-méthyl-4-trifluorométhyl-1(2H)-pyrimidinyl]-benzoyloxy}-propionate d'isopropyle,

le 2-éthyl-2-{2-chloro-5-[3,6-dihydro-2,6-dioxo-3-méthyl-4-trifluorométhyl-1(2H)-pyrimidinyl]-benzoyloxy}-propionate d'éthyle,

le 2-{2-chloro-5-[3,6-dihydro-2,6-dioxo-3-méthyl-4-trifluorométhyl-1(2H)-pyrimidinyl]-benzoyloxy}-2-méthyl-propionate de tert-butyle,

le 2-{2-chloro-5-[3,6-dihydro-2,6-dioxo-3-méthyl-4-trifluorométhyl-1(2H)-pyrimldinyl]-benzoyloxy}-2-méthyl-propionate d'allyle,

le 2-{2-chloro-5-[3,6-dihydro-2,6-dioxo-3-méthyl-4-trifluorométhyl-1(2H)-pyrimidinyl]-benzoyloxy}-2-méthyl-propionate de cyclopentyle,

le 2-{2-chloro-5-[3,6-dihydro-2,6-dioxo-3-méthyl-4-trifluorométhyl-1(2H)-pyrimidinyl]-benzoyloxy}-propionate d'éthyle,

le 2-{2-chloro-5-[3,6-dihydro-2,6-dioxo-3-méthyl-4-trifluorométhyl-1(2H)-pyrimidinyl]-benzoyloxy}-2-méthyl-propionate d'éthyle,

le 2-chloro-5-[3,6-dihydro-2,6-dioxo-3-méthyl-4-trifluorométhyl-1(2H)-pyrimidinyl]-benzoate de 1-acétyléthyle et

le 3-{2-chloro-5-[3,6-dihydro-2,6-dioxo-3-méthyl-4-trifluorométhyl-1(2H)-pyrimidinyl]-benzoyloxy}-butanoate d'éthyle.

ainsi que des adjuvants de formulation.

**12.** Procédé pour la préparation de composés de formule générale

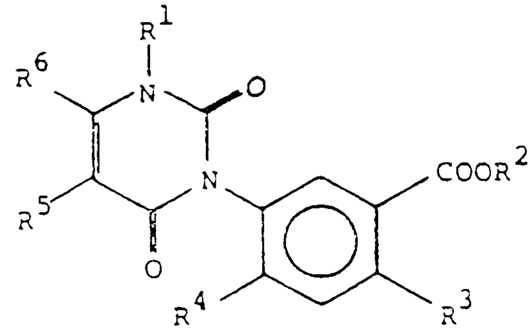

I

dans laquelle

- $R^1$ représente un atome d'hydrogène ou un groupe alkyle en $C_{1-4}$, alcényle en $C_{3\ ou\ 4}$, alcynyle en $C_{3\ ou\ 4}$ ouhalogénoalkyle en $C_{1-4}$,
- $R^2$ représente un groupe alkyl($C_{1-6}$)sulfonyl-alkyle($C_{1-4}$), dialkyl($C_{1-6}$)phosphono-alkyle-($C_{1-4}$), trialkyl($C_{1-6}$)silyl-alkyle($C_{1-4}$), alcanoyl($C_{2-7}$)-alkyle($C_{1-4}$), carboxy-alkyle-($C_{1-4}$), alcoxy($C_{1-6}$)carbonyl-alkyle($C_{1-4}$),{[α-alkylalkylidène($C_{3-9}$)]iminooxy-alcoxy-($C_{1-6}$)carbonyl}-alkyle($C_{1-4}$), alcényloxy($C_{3-6}$)carbonyl-alkyle($C_{1-4}$), alcynyloxy($C_{3-6}$)-carbonyl-alkyle($C_{1-4}$), cycloalkyloxy($C_{3-8}$)carbonyl-alkyle($C_{1-4}$), phénoxy-alkyle($C_{1-4}$), alcényloxy($C_{3-6}$)-alkyle($C_{1-4}$), cycloalcényloxy($C_{3-8}$)-alkyle($C_{1-4}$), cycloalcényle en $C_{5-8}$, bicycloalkyle en $C_{6-8}$, bicycloalcényle en $C_{6-8}$, cyanoalkyle($C_{2-7}$) ou alcynyloxy($C_{3-6}$)-alkyle($C_{1-4}$),
- $R^3$ représente un atome d'halogène ou le groupe cyano,
- $R^4$ représente un atome d'hydrogène ou d'halogène,
- $R^5$ représente un atome d'hydrogène ou de fluor ou un groupe alkyle en $C_{1-4}$, et
- $R^6$ représente un groupe alkyle en $C_{1-4}$ ou halogénoalkyle en $C_{1-4}$, ou
- $R^5$ et $R^6$ forment ensemble un groupe tri- ou tétraméthylène,

et des énoléthers correspondants des composés de formule I dans lesquels $R^1$ est différent d'un atome d'hydrogène ou d'un groupe halogénoalkyle en $C_{1-4}$, ainsi que des sels des composés de formule I dans lesquels $R^1$ représente un atome d'hydrogène, caractérisé en ce que

a) pour préparer les composés de formule I dans lesquels $R^1$ représente un atome d'hydrogène, ainsi que, si on le désire, les sels métalliques de ces composés, on soumet à une cyclisation dans des consitions basiques un composé de formule générale

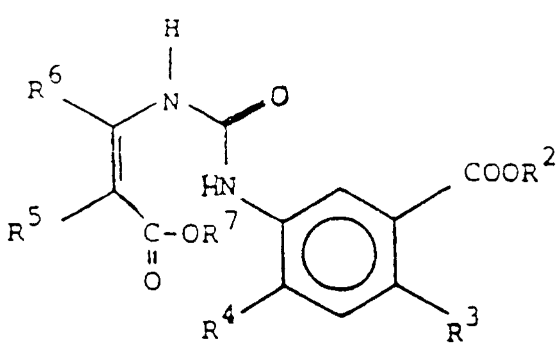

II

dans laquelle $R^2$, $R^3$, $R^4$ , $R^5$ et $R^6$ ont les significations données plus haut, et $R^7$ représente un groupe alkyle inférieur,

et, si on le désire, on convertit en la forme acide ($R^1$ = H) un sel métallique du dérivé d'uracile de formule I, éventuellement obtenu, par traitement par un acide;

b) pour préparer les composés de formule I dans lesquels $R^1$ représente un atome d'hydrogène et $R^6$ est différent d'un groupe halogénoalkyle en $C_{1-4}$, ainsi que, si on le désire, les sels métalliques

de ces composés, on soumet à une cyclisation dans des conditions basiques un composé de formule générale

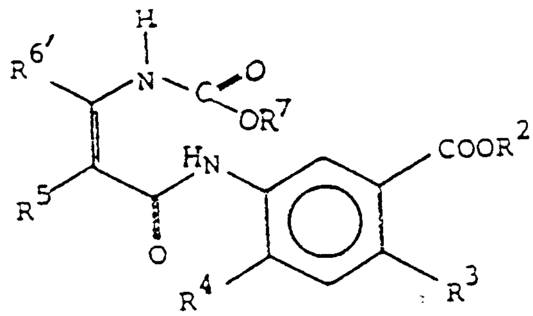

III

dans laquelle $R^2$, $R^3$, $R^4$, $R^5$ et $R^7$ ont les significations données plus haut et $R^{6'}$ représente un groupe alkyle en $C_{1-4}$ ou, conjointement avec $R^5$, un groupe triméthylène ou tétraméthylène,
et si on le désire, on convertit en la forme acide ($R^1$ = H) un sel métallique, éventuellement obtenu, du dérivé d'uracile de formule I, par traitement par un acide;
c) pour préparer les composés de formule I dans lesquels $R^1$ représente un groupe alkyle en $C_{1-4}$, alcényle en $C_{3\text{ ou }4}$, alcynyle en $C_{3\text{ ou }4}$ ou halogénoalkyle en $C_{1-4}$, on soumet à une alkylation avec un agent d'alkylation correspondant, contenant un groupe alkyle en $C_{1-4}$, alcényle en $C_{3\text{ ou }4}$, alcynyle en $C_{3\text{ ou }4}$ ou halogénoalkyle en $C_{1-4}$, un dérivé d'uracile de formule générale

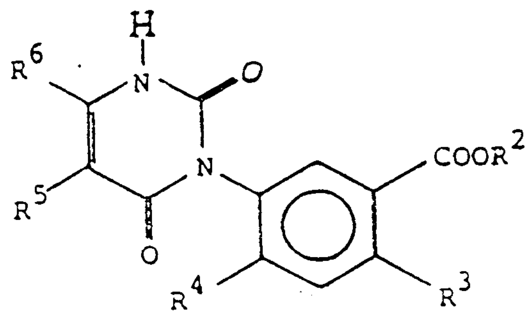

I'

dans laquelle $R^2$, $R^3$, $R^4$, $R^5$ et $R^6$ ont les significations données plus haut;
d) pour préparer les composés de formule I dans lesquels $R^1$ est différent d'un atome d'hydrogène, et les énoléthers correspondants, on estérifie un acide benzoïque de formule générale

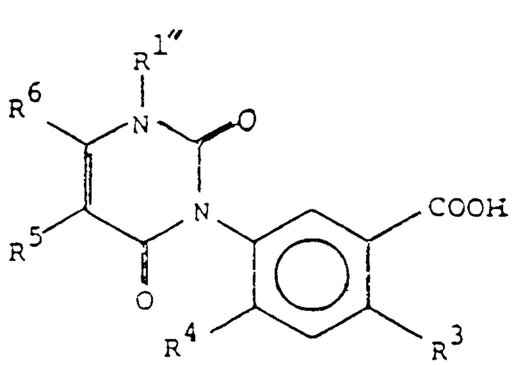

IV

dans laquelle $R^{1''}$ représente un groupe alkyle en $C_{1-4}$, alcényle en $C_{3\text{ ou }4}$, alcynyle en $C_{3\text{ ou }4}$ ou halogénoalkyle en $C_{1-4}$, et $R^3$, $R^4$ , $R^5$ et $R^6$ ont les significations données plus haut, ou l'énoléther correspondant, l'acide benzoïque ou son énoléther pouvant être présent sous forme d'un dérivé réactif,
avec un composé hydroxylé de formule générale

HO-$R^2$ V

dans laquelle $R^2$ a la signification donnée plus haut, ou avec un dérivé réactif de ce composé hydroxylé;

e) pour préparer les composés de formule I dans lesquels $R^1$ est différent d'un atome d'hydrogène, on soumet à une réaction de transestérification un ester d'acide benzoïque de formule générale

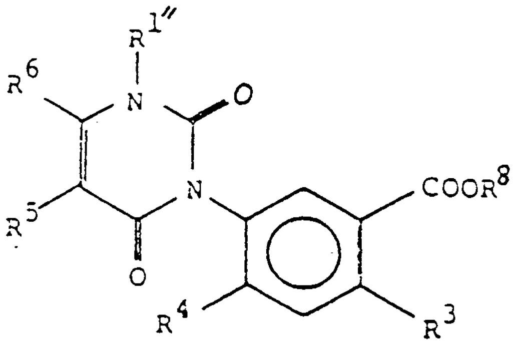

VI

dans laquelle $R^{1''}$, $R^3$, $R^4$, $R^5$ et $R^6$ ont les significations données plus haut et $R^8$ représente un groupe alkyle en $C_{1-6}$, alcényle en $C_{2-4}$, alcynyle en $C_{2-4}$ ou alcoxyalkyle en $C_{2-6}$,

avec un composé hydroxylé de formule V indiquée plus haut, le réactif V ayant un point d'ébullition plus élevé que celui de l'alcanol, de l'alcénol ou de l'alcynol $R^8$OH;

f) pour préparer les composés de formule I dans lesquels $R^3$ représente le groupe cyano, et les énoléthers correspondants, on traite par un cyanure métallique un ester d'acide 2-halogéno-benzoïque de formule générale

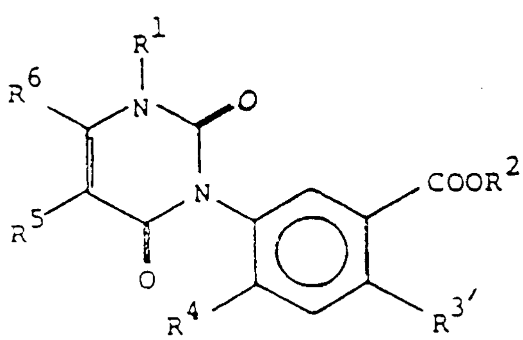

I″

dans laquelle $R^{3'}$ représente un atome d'halogène, et $R^1$, $R^2$, $R^4$, $R^5$ et $R^6$ ont les significations données plus haut,

ou l'énoléther correspondant;

g) pour préparer les énoléthers des composés de formule I, on traite un dérivé d'uracile de formule générale

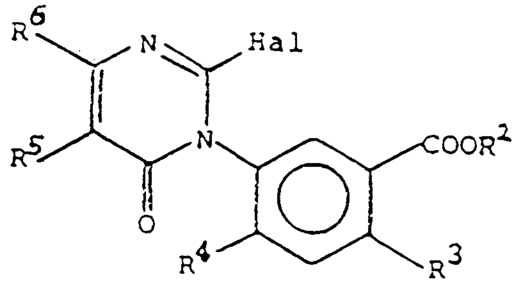

VII

dans laquelle $R^2$, $R^3$, $R^4$, $R^5$ et $R^6$ ont les significations données plus haut et Hal représente un atome de chlore ou de brome,

par un alcanol, alcénol ou alcynol $R^{1'}OH$, en présence d'une base organique, ou par l'alcoolate, l'alcénolate ou l'alcynolate correspondant, de formule générale

$R^{1'}O^{\ominus}M^{\oplus}$ VIII

dans laquelle $R^{1'}$ a la signification donnée plus haut et $M^{\oplus}$ représente un équivalent d'un ion métallique,

et, si on le désire, on convertit en un sel un composé de formule I ainsi obtenu dans lequel $R^1$ représente un atome d'hydrogène.

**13.** Procédé selon la revendication 12, caractérisé en ce que $R^2$ est différent d'un groupe carboxyalkyle-($C_{1-4}$), $R^5$ représente un atome d'hydrogène ou de fluor ou un groupe alkyle en $C_{1-4}$, et $R^6$ représente un groupe alkyle en $C_{1-4}$ ou halogénoalkyle en $C_{1-4}$, et en ce que les composés de formule I et leurs énoléthers et leurs sels sont préparés par la variante a), c), d), e) ou g) du procédé, et éventuellement par la conversion en un sel.

**14.** Procédé selon la revendication 12, caractérisé en ce que l'on prépare un composé selon l'une des revendications 1 à 7.

**15.** Procédé pour la lutte contre les mauvaises herbes, caractérisé en ce que l'on traite les mauvaises herbes et/ou la culture à protéger contre les mauvaises herbes, par une quantité efficace d'un composé selon l'une des revendications 1, 4, 5 et 7 ou d'une composition selon la revendication 8 ou 11.

**16.** Procédé pour la lutte contre les mauvaises herbes, caractérisé en ce que l'on traite les mauvaises herbes et/ou la culture à protéger contre les mauvaises herbes, par une quantité efficace d'un composé selon l'une des revendications 2, 3 et 6 ou d'une composition selon la revendication 9 ou 10.

**17.** Utilisation d'un composé selon l'une des revendications 1, 4, 5 et 7 ou d'une composition selon la revendication 8 ou 11, pour la lutte contre les mauvaises herbes.

**18.** Utilisation d'un composé selon l'une des revendications 2, 3 et 6 ou d'une composition selon la revendication 9 ou 10, pour la lutte contre les mauvaises herbes.